# EUROPEAN PATENT SPECIFICATION

(11) **EP 4 090 766 B1**
(45) Date of publication and mention of the grant of the patent: **21.05.2025**
(21) Application number: 21741739.3
(22) Date of filing: 15.01.2021
(51) Int. Cl.: C12Q 1/6806, C12Q 1/6853, C12Q 1/6876, C12N 9/22

(54) **METHODS OF TARGETED SEQUENCING**
VERFAHREN ZUR GEZIELTEN SEQUENZIERUNG
PROCÉDÉS DE SÉQUENÇAGE CIBLÉ

(30) Priority: 17.01.2020 US 202062962777 P; 14.04.2020 US 202063009603 P
(43) Date of publication of application: 23.11.2022
(60) Divisional application: 25169070.7
(73) Proprietor: Jumpcode Genomics, Inc., San Diego, CA 92121 (US)
(72) Inventor: BROWN, Keith, San Diego, California 92121 (US)
(74) Representative: J A Kemp LLP
(86) International application number: PCT/US2021/013598
(87) International publication number: WO 2021/146534

(56) References cited:
- WO-A1-2016/040524
- WO-A1-2019/178577
- WO-A2-2016/100955
- US-A1- 2017 101 674
- US-A1- 2017 247 689
- US-A1- 2018 010 180
- US-A1- 2018 016 630
- US-A1- 2019 360 044
- XU XINHUI ET AL: "CRISPR-assisted targeted enrichment-sequencing (CATE-seq)", BIORXIV, 17 June 2019 (2019-06-17), XP093096041, Retrieved from the Internet <URL:https://www.biorxiv.org/content/10.1101/672816v1.full.pdf> [retrieved on 20231029], DOI: 10.1101/672816

## Description

This application claims the benefit of U.S. Provisional Application No. 62/962,777, filed January 17, 2020, and U.S. Provisional Application No. 63/009,603, filed April 14, 2020.

### BACKGROUND

Advances in nucleic acid sequencing allow for large numbers of samples to be sequenced at an increasingly affordable price. However, it may be desirable to sequence just one or a handful of target sequences to reduce time and cost of data processing, cost of sequencing or to increase sensitivity of detection for rare events.

WO 2016/040524 A1 discloses methods and compositions for rapid nucleic acid library preparation.

WO 2016/100955 A2 discloses compositions and methods for targeted depletion, enrichment, and partitioning of nucleic acids using CRISPR/Cas system proteins.

WO 2019/178577 A1 discloses methods and reagents for enrichment of nucleic acid material for sequencing applications and other nucleic acid material interrogations.

Xu Xinhui et al. 2019 discloses CRISPR-assisted targeted enrichment-sequencing (CATE-seq).

### SUMMARY

The invention is defined in claim 1. Further aspects and preferred embodiments are defined in the dependent claims. Any aspects, embodiments and examples of the present disclosure which do not fall under the scope of the appended claims do not form part of the invention and are merely provided for illustrative purposes. Thus, the present invention provides a method of creating a nucleic acid library comprising a target nucleic acid, the method comprising: (a) contacting a nucleic acid sample to a first population of primers, a polymerase, dNTPs, and labeled ddNTPs; (b) performing an extension reaction thereby creating an labeled extension product; (c) contacting the extension product to a second population of primers to create a double stranded extension product comprising the target nucleic acid; (d) contacting the double stranded extension product to a target specific enzyme under conditions allowing cleavage of at least a subset of the double stranded extension product thereby creating a cleaved target nucleic acid; and (e) isolating the cleaved target nucleic acid, thereby creating a nucleic acid library. The invention also provides a method of generating a sequencing library comprising a target nucleic acid sequence, the method comprising: (a) incorporating an affinity molecule to a double stranded DNA molecule comprising a target nucleic acid sequence thereby obtaining an affinity molecule-coupled double stranded DNA molecule, (b) capturing the affinity molecule coupled double stranded DNA molecule using a binding agent that binds to the affinity molecule; (c) cutting the target nucleic acid using a CRISPR enzyme mediated cleavage, and (d) isolating the cleaved target nucleic acid from the solid substrate and converting into a sequencing library. Targeted Sequencing panels are an important research and diagnostic tool to enable the focused sequencing and variant discovery of known and unknown molecular events within known gene sequences. Applications include cancer hotspot sequencing, inherited disease, pharmacogenomics, infectious disease and other applications in both human and agriculturally relevant species. The main justification for targeted or locus specific deoxyribonucleic acid (DNA) (and ribonucleic acid (RNA)) sequencing is to reduce cost and increase sensitivity.

A targeted sequencing technology that can eliminate these flaws is thus very attractive. Such a method must have the following characteristics: no fragmentation of template molecules; will work on both RNA and DNA; does not require extensive optimization for each target set; has the sensitivity and speed of polymerase chain reaction (PCR); has the scale and cost of hybrid capture; does not bias results toward pre-determined "reference" sequences; provides uniform coverage of all target loci; and can work from minute sample sources and diverse sample qualities (i.e. highly fragmented or degraded material).

In some embodiments, methods herein combine the proven performance of the Riptide Rapid Library preparation, with the specificity and speed of clustered regularly interspaced short palindromic repeats (CRISPR) based targeting. Sample inputs can be RNA or DNA. Template molecules can be heat denatured and contacted to a random primer with a 5' sample barcode tail. A high fidelity polymerase can be used to extend the primer from the 3' end, making an exact copy of the template sequence. The extended product can be terminated using a small amount of biotinylated dideoxynucleotide triphosphates (ddNTPs). This first step can be used to barcode molecules while simultaneously generating a fragment size amenable to sequencing and adding an affinity molecule for purification. Purification can occur by binding the terminal biotin molecules to streptavidin coated magnetic beads. This approach can allow for the isolation of barcoded products while rendering the molecules incapable of cross hybridization and further 3' extension of other template molecules. This also can allow for multiplexed sample processing at the earliest stage possible. Multiple samples can be pooled into a single reaction tube after this initial barcoding step.

After products are immobilized on beads (or even coated microwells) they can be converted to double stranded molecules through an additional random priming step. In some cases, a high-fidelity strand displacing polymerase is used, meaning that the primer bound closest to the bead can displace all products downstream in this step. The resulting molecule composition can include a double stranded barcode (blunt end) on one side and a double stranded copy of the template bound to beads. In some cases, an alternative to a random primer is a combination of primase along with a strand displacing polymerase to produce this product. Multiple samples can now exist in this format, all with unique sample barcodes generated from the first priming step in the reaction. All excess reactants can be washed away. To enrich for targeted loci, a pool of guide RNA molecules targeting adjacent sequences to the exons, single nucleotide variants (SNVs) or other gene targets of interest can be designed and synthesized either directly or through in vitro transcription of DNA templates. The pool of guide RNA molecules can be incubated with CAS9 or a CAS enzyme derivative to form a multiplexed pool of ribo-nucleoprotein (RNP) complexes. These RNP complexes can then be incubated with the captured products previously described, making a double stranded cut of the target molecules, which are eluted from the beads. Nontarget molecules can remain bound to the beads. The resulting pool (elution) can contain blunt end target sequences from a multiplex sample library containing a sample barcode on one end and a blunt end target site corresponding to the CRISPR RNA target on the other end. These blunt end products can then be subjected to standard library construction through ligation of universal adapters. In some cases, plate specific barcodes are added (in the index read position) to enable greater multiplexing of samples and further reduced costs.

This method can be used in applications such as: cancer hotspots/ liquid biopsies; gene specific NIPT; inherited disease/ autosomal recessive gene targets (Tay Sachs, Cystic Fibrosis, etc.); 16s identification of microbes; pharmacogenomic / absorption, distribution, metabolism and excretion (ADME) gene targets; infectious disease targets; forensic markers; and customized to any target sites.

In some cases, this approach enables multiple testing of gene targets or confirmation of genotypes. For example, low pass sequencing and imputation can be used to perform a genome-wide association study (GWAS) and confirmation testing can occur on the sample library for deeper coverage of candidate SNVs or candidate gene sequences without the requirement to prepare a second set of libraries. The same barcoded sample sets can be used for serial testing of different gene targets as new discoveries are made and need a low cost confirmation.

The invention provides methods of creating a nucleic acid library comprising a target nucleic acid. In some cases, the method comprises: (a) contacting a nucleic acid sample to a first population of primers, a polymerase, deoxynucleotide triphosphates (dNTPs), and labeled ddNTPs; (b) performing an extension reaction thereby creating an labeled extension product; (c) contacting the extension product to a second population of primers to create a double stranded extension product; (d) contacting the double stranded extension product to a target specific enzyme under conditions allowing cleavage of at least a subset of the double stranded extension product thereby creating a cleaved target nucleic acid; and (e) isolating the cleaved target nucleic acid, thereby creating a nucleic acid library. In some cases, the nucleic acid sample is denatured. In some cases, the first or second population of primers comprise random primers. In some cases, the first or second population of primers comprise a barcode. In some cases, the first or second population of primers comprise oligo-dT primers. In some cases, the labeled ddNTPs comprise biotin. In some cases, the labeled extension product is isolated on a bead. In some cases, the bead is a streptavidin bead. In some cases, the bead is a magnetic bead. In some cases, the labeled extension product is isolated on a polycarbonate, polypropylene, or polycarbonate surface. In some cases, the target specific enzyme is complexed with a target specific guide RNA. In some cases, the target specific enzyme is a CRISPR enzyme. In some cases, the target specific enzyme is a Cas nuclease. In some cases, the target specific enzyme is a Cas9 nuclease. In some cases, the target specific enzyme is a nuclease. In some cases, the target nucleic acid is a deoxyribonucleic acid. In some cases, the target nucleic acid is a ribonucleic acid. In some cases, the polymerase is a DNA polymerase, an RNA polymerase, a reverse transcriptase, or a combination thereof. In some cases, the target nucleic acid comprises a viral nucleic acid, a bacterial nucleic acid, a single nucleotide polymorphism, a disease associated gene, a cancer associated gene, a human leukocyte antigen gene, or a combination thereof. In some cases, the virus is an influenza virus, a coronavirus, a rhinovirus, a herpesvirus, or a human immunodeficiency virus. In some cases, the coronavirus is a COVID-19 virus. In some cases, the target nucleic acid is about 5 kb to about 20 kb. In some cases, the target nucleic acid is about 10 kb to about 15 kb.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

An understanding of the features and advantages of the present invention will be obtained by reference to the following detailed description that sets forth illustrative embodiments, in which the principles of the invention are utilized, and the accompanying drawings of which:
**FIG. 1** illustrates a targeted sequencing method.
**FIG. 2** illustrates a single tube HCoV2 targeted sequencing method.
**FIG. 3** illustrates targeted sequencing of an E. *coli* genome.
**FIG. 4** illustrates long and short read applications of targeted sequencing.
**FIG. 5** illustrates adapter and barcode applications of targeted sequencing.
**FIG. 6** illustrates multiple target molecule generation.
**FIG. 7** illustrates small fragments for sequence ready library generation.

### DETAILED DESCRIPTION

The most commonly used targeted sequencing approaches include PCR or PCR-like derivatives and hybridization-based capture protocols. Both techniques were developed long before the first massively parallel sequencers were commercialized. Dramatic progress has been made in the speed and cost of these approaches over the past two decades, however they have likely reached the limits of innovation. Thus, new technologies are needed to replace these antiquated approaches to targeted sequencing as both require expensive and time-consuming optimization.

PCR and PCR derivatives have the benefit of a relatively fast sample processing time, along with good specificity and sensitivity. However, these approaches are expensive, require extensive optimization, and require cumbersome post amplification normalization when multiplexing. In addition, these approaches, when using two opposing strand primers, have a high dropout rate, do not allow for redundant sampling of the same locus and lack the ability to detect or discover novel rearrangements. Clonal amplification of polymerase errors is also a major issue. Strategies to overcome some of these limitations include the use of Unique Molecular Indices (UMIs) or barcodes to detect clonal polymerase errors as well as hemi-specific or "nested" approaches to detect rearrangements. The latter requiring library preparation using fragmentation prior to the hemi-specific approach. These methods also require a lot of optimization and are not meant for long read sequencing systems. Further, single nucleotide polymorphisms (SNPs) under primer sites often cause dropouts. In addition, allelic bias is a problem, along with lack in uniformity of coverage (representation of each amplicon).

Hybridization based approaches, called hybrid capture, offer the ability to discover novel structural rearrangements and offer increased scale in terms of the number of targets that can be sequenced simultaneously. However, there are still numerous flaws with hybrid capture approaches. To date, all hybrid capture approaches start with a fragmentation step. This step produces loss of information as template molecules will be lost due to the damage caused by chemical or physical fragmentation. At best, 60% of an individual nucleic acid molecule is retained after the most efficient fragmentation protocols. In addition, hybrid capture approaches have documented reference allele bias for the sequence to which the hybridization probes are initially designed. Capture efficiencies vary widely due to differences in sequence content and therefore require over sequencing of samples in order to reach a minimum coverage needed for clinical grade sequencing.

In fact, a January 2020 publication by researchers at the University of Texas Southwestern found that three of the leading commercial exome sequencing providers fail to cover critical genes. "A review of clinical tests from three major U.S. laboratories shows whole exome sequencing routinely fails to adequately analyze large segments of DNA, a potentially critical deficiency that can prevent doctors from accurately diagnosing potential genetic disorders, from epilepsy to cancer. The reanalysis by UT Southwestern shows each lab on average adequately examined less than three-quarters of the genes -- 34, 66, and 69 percent coverage -- and had startlingly wide gaps in their ability to detect specific disorders. Researchers say they conducted the study because they believe vast differences in testing quality are endemic in clinical genetic sequencing but have not been well documented or shared with clinicians.

### (source: https:/ Iwww.sciencedaily.com/releases/2020101/200106103451.htm)

Hybrid capture strategies allow for increased multiplexing (larger number of targets) and avoid clonal amplification errors. However, workflows are long and cumbersome and capture is not very specific. This method also suffers from allelic bias and dropout and is not ideal for long read sequencing.

An ideal targeted sequencing strategy would allow for unlimited multiplexing, uniformity of coverage/ representation of all targets in the sample, and would work on long read and short read sequencing technology. It would also have a high conversion rate on initial designs.

Currently, targeted sequencing technologies are mainly either amplicon based or hybridization based. Amplicon based strategies generally use PCR or PCR derivatives. These can be sensitive and relatively fast protocols. However, they often have limited multiplexing ability due to primer design constraints (i.e. no overlapping amplicons). It also generally requires a lot of optimization and are not meant for long read sequencing systems. Further, SNPs under primer sites can cause dropouts. Additionally, clonal polymerase errors and allelic bias can be a problem. Another issue in amplification based targeted sequencing is uniformity of coverage (i.e., representation of each amplicon).

Hybridization capture strategies can allow for increased multiplex (larger number of targets) and avoid clonal amplification errors. However, workflows can be long and cumbersome. Specificity, allelic bias, and dropout and also pose issues in this method. Hybridization methods are also not ideal for long read sequencing.

An ideal targeted sequencing strategy would allow for unlimited multiplexing, provide uniformity of coverage and representation of all targets in the sample, would work on long read and short read sequencing technology and have a high conversion rate on initial designs.

### Definitions

A partial list of relevant definitions is as follows.

"Amplified nucleic acid" or "amplified polynucleotide" as used herein is any nucleic acid or polynucleotide molecule whose amount has been increased at least two fold by any nucleic acid amplification or replication method performed *in vitro* as compared to its starting amount. For example, an amplified nucleic acid is obtained from a polymerase chain reaction (PCR) which can, in some instances, amplify DNA in an exponential manner (for example, amplification to 2ⁿ copies in n cycles). Amplified nucleic acid can also be obtained from a linear amplification.

"Amplification product" as used herein can refer to a product resulting from an amplification reaction such as a polymerase chain reaction.

An "amplicon" as used herein is a polynucleotide or nucleic acid that is the source and/or product of natural or artificial amplification or replication events.

The term "biological sample" or "sample" as used herein generally refers to a sample or part isolated from a biological entity. The biological sample may show the nature of the whole and examples include, without limitation, bodily fluids, dissociated tumor specimens, cultured cells, and any combination thereof. Biological samples can come from one or more individuals. One or more biological samples can come from the same individual. One non limiting example would be if one sample came from an individual's blood and a second sample came from an individual's tumor biopsy. Examples of biological samples can include but are not limited to, blood, serum, plasma, nasal swab or nasopharyngeal wash, saliva, urine, gastric fluid, spinal fluid, tears, stool, mucus, sweat, earwax, oil, glandular secretion, cerebral spinal fluid, tissue, semen, vaginal fluid, interstitial fluids, including interstitial fluids derived from tumor tissue, ocular fluids, spinal fluid, throat swab, breath, hair, finger nails, skin, biopsy, placental fluid, amniotic fluid, cord blood, emphatic fluids, cavity fluids, sputum, pus, microbiota, meconium, breast milk and/or other excretions. The samples may include nasopharyngeal wash. Examples of tissue samples of the subject may include but are not limited to, connective tissue, muscle tissue, nervous tissue, epithelial tissue, cartilage, cancerous or tumor sample, or bone. The sample may be provided from a human or animal. The sample may be provided from a mammal, including vertebrates, such as murines, simians, humans, farm animals, sport animals, or pets. The sample may be collected from a living or dead subject. The sample may be collected fresh from a subject or may have undergone some form of pre-processing, storage, or transport.

"Bodily fluid" as used herein generally can describe a fluid or secretion originating from the body of a subject. In some instances, bodily fluids are a mixture of more than one type of bodily fluid mixed together. Some non-limiting examples of bodily fluids are: blood, urine, bone marrow, spinal fluid, pleural fluid, lymphatic fluid, amniotic fluid, ascites, sputum, or a combination thereof.

"Complementary" or "complementarity" as used herein can refer to nucleic acid molecules that are related by base-pairing. Complementary nucleotides are, generally, A and T (or A and U), or C and G (or G and U). Two single stranded RNA or DNA molecules are said to be substantially complementary when the nucleotides of one strand, optimally aligned and with appropriate nucleotide insertions or deletions, pair with at least about 90% to about 95% complementarity, and more preferably from about 98% to about 100%) complementarity, and even more preferably with 100% complementarity. Alternatively, substantial complementarity exists when an RNA or DNA strand will hybridize under selective hybridization conditions to its complement. Selective hybridization conditions include, but are not limited to, stringent hybridization conditions. Hybridization temperatures are generally at least about 2° C to about 6° C lower than melting temperatures (Tₘ).

A "barcode" or "molecular barcode" as used herein is a material for labeling. The barcode can label a molecule such as a nucleic acid or a polypeptide. The material for labeling is associated with information. A barcode can be called a sequence identifier (i.e. a sequence-based barcode or sequence index). A barcode can be a particular nucleotide sequence. A barcode can be used as an identifier. A barcode can be a different size molecule or different ending points of the same molecule. Barcodes can include a specific sequence within the molecule and a different ending sequence. For example, a molecule that is amplified from the same primer and has 25 nucleotide positions is different than a molecule that is amplified and has 27 nucleotide positions. The addition positions in the 27mer sequence can be considered a barcode. A barcode can be incorporated into a polynucleotide. A barcode can be incorporated into a polynucleotide by many methods. Some non-limiting methods for incorporating a barcode can include molecular biology methods. Some non-limiting examples of molecular biology methods to incorporate a barcode are through primers *(e.g.,* tailed primer elongation), probes *(i.e.,* elongation with ligation to a probe), or ligation *(i.e.,* ligation of known sequence to a molecule).

A barcode can be incorporated into any region of a polynucleotide. The region can be known. Alternatively, the region can be unknown. The barcode can be added to any position along the polynucleotide. In some cases, the barcode can be added to the 5' end of a polynucleotide. Alternatively, the barcode can be added to the 3' end of the polynucleotide. The barcode can be added in between the 5' and 3' end of a polynucleotide. In some cases, the barcode is added with one or more other known sequences. One non-limiting example is the addition of a barcode with a sequence adapter.

Barcodes can be associated with information. Some non-limiting examples of the type of information a barcode is associated with information include: the source of a sample; the orientation of a sample; the region or container a sample was processed in; the adjacent polynucleotide; or any combination thereof.

**In** some cases, barcodes are made from combinations of sequences (different from combinatorial barcoding) and is used to identify a sample or a genomic coordinate and a different template molecule or single strand the molecular label and copy of the strand was obtained from. In some cases, a sample identifier, a genomic coordinate, and a specific label for each biological molecule can be amplified together. Barcodes, synthetic codes, or label information can also be obtained from the sequence context of the code (allowing for errors or error correcting), the length of the code, the orientation of the code, the position of the code within the molecule, and in combination with other natural or synthetic codes.

Barcodes can be added before pooling of samples. When the sequences are determined of the pooled samples, the barcode can be sequenced along with the rest of the polynucleotide. In some cases, the barcode is used to associate the sequenced fragment with the source of the sample.

Barcodes can also be used to identify the strandedness of a sample. One or more barcodes can be used together. Two or more barcodes can be adjacent to one another, not adjacent to one another, or any combination thereof.

**In** some cases, barcodes are used for combinatorial labeling.

"Combinatorial labeling" as used herein is a method by which two or more barcodes are used to label. The two or more barcodes can label a polynucleotide. The barcodes, each, alone is associated with information. The combination of the barcodes together can be associated with information. In some cases, a combination of barcodes is used together to determine in a randomly amplified molecule that the amplification occurred from the original sample template and not a synthetic copy of that template. In some cases, the length of one barcode in combination with the sequence of another barcode is used to label a polynucleotide. In some cases, the length of one barcode in combination with the orientation of another barcode is used to label a polynucleotide. In other cases, the sequence of one barcode is used with the orientation of another barcode to label a polynucleotide. In some cases, the sequence of a first and a second bar code, in combination with the distance in nucleotides between them, is used to label or to identify a polynucleotide.

"Double-stranded" as used herein can refer to two polynucleotide strands that have annealed through complementary base-pairing.

"Known oligonucleotide sequence" or "known oligonucleotide" or "known sequence" as used herein can refer to a polynucleotide sequence that is known. A known oligonucleotide sequence can correspond to an oligonucleotide that has been designed, *e.g.,* a universal primer for next generation sequencing platforms (e.g., Illumina, 454), a probe, an adaptor, a tag, a primer, a molecular barcode sequence, an identifier. A known sequence can comprise part of a primer. A known oligonucleotide sequence may not actually be known by a particular user but is constructively known, for example, by being stored as data which may be accessible by a computer. A known sequence may also be a trade secret that is actually unknown or a secret to one or more users but may be known by the entity who has designed a particular component of the experiment, kit, apparatus or software that the user is using.

"Library" as used herein can refer to a collection of nucleic acids. A library can contain one or more target fragments. In some instances the target fragments is amplified nucleic acids. In other instances, the target fragments is nucleic acid that is not amplified. A library can contain nucleic acid that has one or more known oligonucleotide sequence(s) added to the 3' end, the 5' end or both the 3' and 5' end. The library may be prepared so that the fragments can contain a known oligonucleotide sequence that identifies the source of the library (e.g., a molecular identification barcode identifying a patient or DNA source). In some instances, two or more libraries is pooled to create a library pool. Libraries may also be generated with other kits and techniques such as transposon mediated labeling, or "tagmentation" as known in the art. Kits may be commercially available, such as the Illumina NEXTERA kit (Illumina, San Diego, CA).

"Locus specific" or "loci specific" as used herein can refer to one or more loci corresponding to a location in a nucleic acid molecule (e.g., a location within a chromosome or genome). In some instances, a locus is associated with genotype. In some instances loci may be directly isolated and enriched from the sample, e.g., based on hybridization and/or other sequence-based techniques, or they may be selectively amplified using the sample as a template prior to detection of the sequence. In some instances, loci may be selected on the basis of DNA level variation between individuals, based upon specificity for a particular chromosome, based on CG content and/or required amplification conditions of the selected loci, or other characteristics that will be apparent to one skilled in the art upon reading the present disclosure. A locus may also refer to a specific genomic coordinate or location in a genome as denoted by the reference sequence of that genome.

"Long nucleic acid" as used herein can refer to a polynucleotide longer than 1, 2, 3, 4, 5, 6, 7, 8, 9, or 10 kilobases.

The term "melting temperature" or "Tₘ" as used herein commonly refers to the temperature at which a population of double-stranded nucleic acid molecules becomes half dissociated into single strands. Equations for calculating the Tₘ of nucleic acids are well known in the art. One equation that gives a simple estimate of the Tₘ value is as follows: Tₘ=81.5+16.6(log 10[Na⁺])0.41(%[G+C])-675/n-1.0 m, when a nucleic acid is in aqueous solution having cation concentrations of 0.5 M or less, the (G+C) content is between 30% and 70%, n is the number of bases, and m is the percentage of base pair mismatches (see, *e.g.,* Sambrook J et al., Molecular Cloning, A Laboratory Manual, 3rd Ed., Cold Spring Harbor Laboratory Press (2001)). Other references can include more sophisticated computations, which take structural as well as sequence characteristics into account for the calculation of Tₘ.

"Nucleotide" as used herein can refer to a base-sugar-phosphate combination. Nucleotides are monomeric units of a nucleic acid sequence (*e.g.,* DNA and RNA). The term nucleotide includes naturally and non-naturally occurring ribonucleoside triphosphates ATP, TTP, UTP, CTG, GTP, and ITP, for example and deoxyribonucleoside triphosphates such as dATP, dCTP, dITP, dUTP, dGTP, dTTP, or derivatives thereof. Such derivatives can include, for example, [aS]dATP, 7-deaza-dGTP and 7-deaza-dATP, and, for example, nucleotide derivatives that confer nuclease resistance on the nucleic acid molecule containing them. The term nucleotide as used herein also refers to dideoxyribonucleoside triphosphates (ddNTPs) and their derivatives. Illustrative examples of dideoxyribonucleoside triphosphates include, ddATP, ddCTP, ddGTP, ddITP, ddUTP, ddTTP, for example. Other ddNTPs are contemplated and consistent with the disclosure herein, such as dd (2-6 diamino) purine.

"Polymerase" as used herein can refer to an enzyme that links individual nucleotides together into a strand, using another strand as a template.

"Polymerase chain reaction" or "PCR" as used herein can refer to a technique for replicating a specific piece of selected DNA *in vitro,* even in the presence of excess non-specific DNA. Primers are added to the selected DNA, where the primers initiate the copying of the selected DNA using nucleotides and, typically, Taq polymerase or the like. By cycling the temperature, the selected DNA is repetitively denatured and copied. A single copy of the selected DNA, even if mixed in with other, random DNA, is amplified to obtain thousands, millions, or billions of replicates. The polymerase chain reaction is used to detect and measure very small amounts of DNA and to create customized pieces of DNA.

The terms "polynucleotides" and "oligonucleotides" as used herein may include but is not limited to various DNA, RNA molecules, derivatives or combination thereof. These may include species such as dNTPs, ddNTPs, 2-methyl NTPs, DNA, RNA, peptide nucleic acids, cDNA, dsDNA, ssDNA, plasmid DNA, cosmid DNA, chromosomal DNA, genomic DNA, viral DNA, bacterial DNA, mtDNA (mitochondrial DNA), mRNA, rRNA, tRNA, nRNA, siRNA, snRNA, snoRNA, scaRNA, microRNA, dsRNA, ribozyme, riboswitch and viral RNA. "Oligonucleotides," generally, are polynucleotides of a length suitable for use as primers, generally about 6-50 bases but with exceptions, particularly longer, being not uncommon.

A "primer" as used herein generally refers to an oligonucleotide used to prime nucleotide extension, ligation and/or synthesis, such as in the synthesis step of the polymerase chain reaction or in the primer extension techniques used in certain sequencing reactions. A primer may also be used in hybridization techniques as a means to provide complementarity of a locus to a capture oligonucleotide for detection of a specific nucleic acid region.

"Primer extension product" as used herein generally refers to the product resulting from a primer extension reaction using a contiguous polynucleotide as a template, and a complementary or partially complementary primer to the contiguous sequence.

"Sequencing," "sequence determination," and the like as used herein generally refers to any and all biochemical methods that may be used to determine the order of nucleotide bases in a nucleic acid.

A "sequence" as used herein refers to a series of ordered nucleic acid bases that reflects the relative order of adjacent nucleic acid bases in a nucleic acid molecule, and that can readily be identified specifically though not necessarily uniquely with that nucleic acid molecule. Generally, though not in all cases, a sequence requires a plurality of nucleic acid bases, such as 5 or more bases, to be informative although this number may vary by context. Thus a restriction endonuclease may be referred to as having a 'sequence' that it identifies and specifically cleaves even if this sequence is only four bases. A sequence need not 'uniquely map' to a fragment of a sample. However, in most cases a sequence must contain sufficient information to be informative as to its molecular source.

As used herein, a sequence 'does not occur' in a sample if that sequence is not contiguously present in the entire sequence of the sample. Sequence that does not occur in a sample is not naturally occurring sequence in that sample.

As used herein, a library is described as "representative of a sample" if the library comprises an informative sequence of the sample. In some cases an informative sequence comprises about 5%, 10%, 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99%, or 100% of a sample sequence. In some cases an informative sequence comprises about 90%, 90%, or greater than 90% of a sample sequence.

As used herein, a sequence or sequence length is described as 'independently determined' if the sequence or sequence length is not determined by or a function of a second sequence or sequence length. Random events such as incorporation of a terminating ddNTP base or nonspecific or less than exact annealing of an oligo to a template are generally events that are independently determined, such that a library of molecules resulting from such events comprises substantial variation in sequence or sequence length.

As used herein, a sequence is described as 'indeterminate' if it is not determined by template-mediated synthesis. Thus a nucleic acid molecule originating from synthesis off of a template primed by annealing to the template of a random oligomer may comprise a region of template-directed sequence resulting from the template-driven nucleic acid extension, and an 'indeterminate sequence' corresponding to the oligomer sequence providing the 3' OH group from which template-driven extension reaction builds. In some cases the oligonucleotide annealing is imperfect, such that the oligomer sequence is not the exact reverse complement of the molecule to which it binds.

"Subdividing" as used herein in the context of a sample sequence refers to breaking a sequence into subsequences, each of which remains a sequence as defined herein. In some instances subdividing and fractionating are used interchangeably.

As used herein, a "contig" refers to a nucleotide sequence that is assembled from two or more constituent nucleotide sequences that share common or overlapping regions of sequence homology. For example, the nucleotide sequences of two or more nucleic acid fragments is compared and aligned in order to identify common or overlapping sequences. Where common or overlapping sequences exist between two or more nucleic acid fragments, the sequences (and thus their corresponding nucleic acid fragments) is assembled into a single contiguous nucleotide sequence.

The term "biotin," as used herein, is intended to refer to biotin (5-[(3aS,4S,6aR)-2-oxohexahydro-1H-thieno[3,4-d]imidazol-4-yl]pentanoic acid) and any biotin derivatives and analogs. Such derivatives and analogs are substances which form a complex with the biotin binding pocket of native or modified streptavidin or avidin. Such compounds include, for example, iminobiotin, desthiobiotin and streptavidin affinity peptides, and also include biotin-.epsilon.-N-lysine, biocytin hydrazide, amino or sulfhydryl derivatives of 2-iminobiotin and biotinyl-ε-aminocaproic acid-N-hydroxysuccinimide ester, sulfo-succinimide-iminobiotin, biotinbromoacetylhydrazide, p-diazobenzoyl biocytin, 3-(N-maleimidopropionyl) biocytin. "Streptavidin" can refer to a protein or peptide that can bind to biotin and can include: native egg-white avidin, recombinant avidin, deglycosylated forms of avidin, bacterial streptavidin, recombinant streptavidin, truncated streptavidin, and/or any derivative thereof.

A "subject" as used herein generally refers to an organism that is currently living or an organism that at one time was living or an entity with a genome that can replicate. The methods, kits, and/or compositions of the disclosure is applied to one or more single-celled or multi-cellular subjects, including but not limited to microorganisms such as bacterium and yeast; insects including but not limited to flies, beetles, and bees; plants including but not limited to corn, wheat, seaweed or algae; and animals including, but not limited to: humans; laboratory animals such as mice, rats, monkeys, and chimpanzees; domestic animals such as dogs and cats; agricultural animals such as cows, horses, pigs, sheep, goats; and wild animals such as pandas, lions, tigers, bears, leopards, elephants, zebras, giraffes, gorillas, dolphins, and whales. The methods of this disclosure can also be applied to germs or infectious agents, such as viruses or virus particles or one or more cells that have been infected by one or more viruses.

A "support" as used herein is solid, semisolid, a bead, a surface. The support is mobile in a solution or is immobile.

The term "unique identifier" as used herein may include but is not limited to a molecular bar code, or a percentage of a nucleic acid in a mix, such as dUTP.

"Repetitive sequence" as used herein refers to sequence that does not uniquely map to a single position in a nucleic acid sequence data set. Some repetitive sequence is conceptualized as integer or fractional multiples of a repeating unit of a given size and exact or approximate sequence.

A "primer" as used herein refers to an oligonucleotide that anneals to a template molecule and provides a 3' OH group from which template-directed nucleic acid synthesis can occur. Primers comprise unmodified deoxynucleic acids in many cases, but in some cases comprise alternate nucleic acids such as ribonucleic acids or modified nucleic acids such as 2' methyl ribonucleic acids.

As used herein, a nucleic acid is double-stranded if it comprises hydrogen-bonded base pairings. Not all bases in the molecule need to be base-paired for the molecule to be referred to as double-stranded.

The term "about" as used herein in reference to a number refers to that number plus or minus up to 10% of that number. The term used in reference to a range refers to a range having a lower limit as much as 10% below the stated lower limit, and an upper number up to 10% above the stated limit.

Provided herein are targeted sequencing methods. Briefly, in some cases, double stranded DNA or cDNA products are bound to a solid substrate. CRISPR CAS systems can be used to "cut" the target molecules, which can then be physically separated from the background molecules that remain bound to the solid substrate. The target molecules can then be converted into a sequencing library and sequenced.

In some RNA targeted sequencing methods, total RNA can be obtained from a sample. In some cases, random primers are bound to the sample RNA templates. Reverse transcriptase can be used to create a cDNA (copy DNA) of the RNA template. In some cases, a mix of dNTP and biotin ddNTP is used in the extension reaction, in some cases terminating cDNA products with biotin ddNTP. In some cases, products are then captured on streptavidin coated magnetic beads and excess reactants are washed away. In some cases, a second strand is synthesized on the captured molecules using a random primer and a strand displacing polymerase. Primers bound closest to the beads can displace products downstream and a double stranded cDNA product remains bound to beads. CRISPR guide RNAs can be designed to recognize target sequences of interest and the bead bound products are subjected to RNA guided cleavage. In some cases, the CRISPR cut products are separated from the bead bound background through magnetic separation and elution. Double stranded cDNA products can be eluted from the beads and converted into a sequencing library.

In some DNA targeted sequencing methods, the same method as above is used. In some cases, the double stranded DNA products are denatured and a DNA polymerase is used to prepare the templates.

In some cases, RNA and DNA are prepared simultaneously with polymerase enzymes capable of using RNA and DNA templates.

### Targeted Sequencing Methods

In additional aspects, there are provided targeted sequencing methods which use the standard RIPTIDE A and B reactions. In this method, DNA samples (or cDNA) are copied with a polymerase using a barcoded random primer, terminated with biotin-ddNTP. In some cases, samples are pooled (96 or more) and captured on streptavidin coated magnetic beads. In some cases, a second strand is formed via strand displacing polymerase. In some cases, active CRISPR-CAS is targeted through sgRNA and cleaves the targeted molecules off of the beads. In some cases, the molecules are eluted and a library is generated with a standard ligation based protocol.

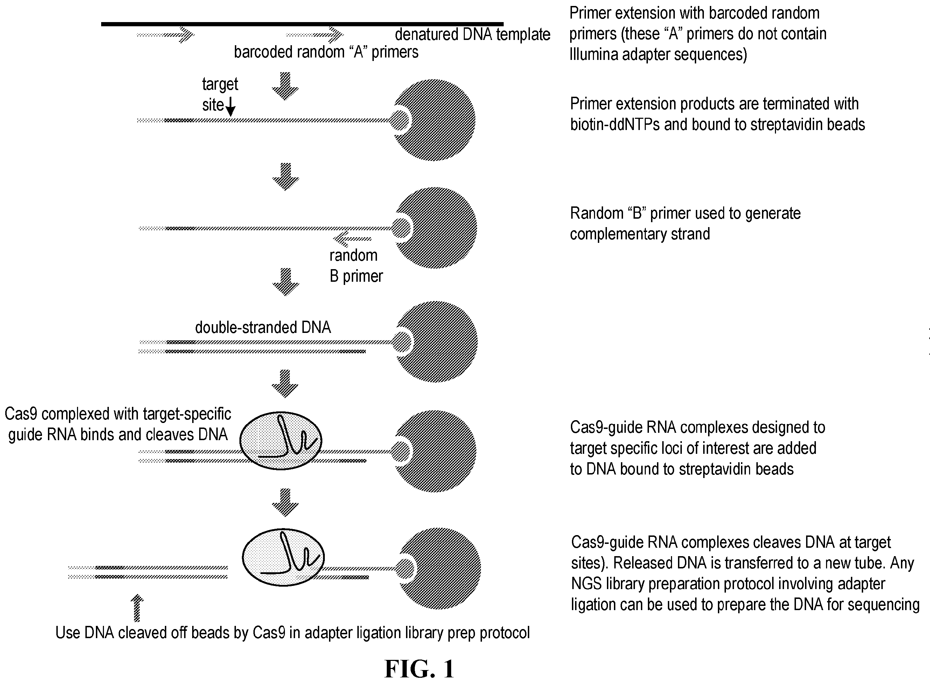

Illustrated in **FIG. 1** is another targeted sequencing method provided herein. In **FIG. 1** primer extension is performed with barcoded random primers (these "A" primers do not contain adapter sequences, such as Illumina adapter sequences). Primer extension products are terminated with biotin ddNTPs and bound to streptavidin beads. Random "B" primers are then used to generate a complementary strand forming a double stranded DNA. Cas9-guide RNA complexes that are designed to target specific loci of interest are added to the DNA bound to streptavidin beads. The Cas9-guide RNA complex cleaves DNA at target sites. The released DNA is then transferred to a new tube. Any suitable NGS library preparation protocol involving adapter ligation can then be used to prepare the DNA for sequencing.

In some methods of targeted sequencing provided herein, an enzyme, such as nickase, can be used to expose 3' ends of molecules in the initial step, then a polymerase, such as a strand displacing polymerase, can extend from the enzyme cleaved site and incorporate affinity molecule, such as biotin. In some cases, RNA molecules can be polyadenylated using biotin dATPs and captured the same way on streptavidin beads or oligodT captured where the oligodT capture oligo can be used as the primer for the second strand synthesis. Any suitable method of capturing a double stranded molecule (preferably at the end) is contemplated to be used to make the intermediate construct. The key is the cutting the target molecules using CRISPR and physically separating them from the solid substrate bound molecules.

In some cases, dsDNA is fragmented, end repaired, A-tailed and ligated to biotinylated adapters. In some cases, biotinylated primers are ligated to 3' end of RNA molecules. In some cases, dsDNA is tagmented with biotinylated DNA. In some cases, single stranded or dsDNA is biotinylated with terminal transferase and biotin labeled dNTPs or ddNTPs. In some cases, 5' end: incorporation during oligonucleotide synthesis, for example, primers used for primer extension, Nextera transposon ends, TSOs, and others. In some cases, 5' end incorporation of biotin-conjugated to a phosphate by kinase treatment is used. In some cases, 3' end incorporation is done by polymerase with 3'-5' exonuclease activity, such as Klenow, where the enzyme would remove the last few 3' nucleotides of a dsDNA molecule and add in biotin-conjugated nucleotides. In some cases, 3' end incorporation uses a biotin-conjugated dideoxynucleotide by treatment with Terminal Transferase. In some cases, 3' end incorporation uses a biotin-conjugated dideoxynucleotide nucleotides during primer extension. In some cases, 5' or 3' end uses incorporation by ligation of an adapter with biotin-nucleotides. In some cases, 5' or 3' end: incorporation uses a biotin-oligo by conjugation chemistry, such as CLICK chemistry.

In some methods of targeted sequencing provided herein, biotin incorporation into the middle of molecules can take place in one of these ways: incorporation of nucleotides during primer extension from an annealed primer; or incorporation of nucleotides into an existing dsDNA template by nicking, then primer extension from the 3' end of the nick with a strand-displacing polymerase, such as Sequenase.

In some methods of targeted sequencing provided herein, barcoded random primers and adapter tailed barcoded random primers can be used in the initial polymerase extension reaction to multiplex samples or to streamline the library preparation workflow.

In some methods of targeted sequencing provided herein, library prep from the CRISPR cleaved products uses any suitable preparation, including but not limited to tagmentation, fragmentation, A tail and ligation, or other suitable methods. For example, double stranded genomic DNA can be tagmented with biotinylated transposons. This would ensure all molecules are relatively the same length when captured.

In exemplary embodiments of targeted sequencing methods herein, a nucleic acid molecule comprising primer 5'-Illumina adapter-8bp sample barcode-random primer-3' is used. Multiple samples (such as individual wells of a microtiter plate) can be processed in the first step (extension and biotin ddNTP termination), then pooled together for capture and wash. Second strand synthesis and CRISPR targeting can occur on all samples simultaneously. In this case, adapters must be added to the other end of the CRISPR cleaved products such as through A-tailing and ligation. Ligation on both ends is prevented using any suitable method such as blocking the products on the beads/solid substrate prior to CRISPR cleavage. When products are cleaved off of the beads, adapters can only ligate to the blunt end CRISPR cut location. PCR enrichment is used to ensure that products with two adapters are enriched for sequencing.

In additional embodiments of targeted sequencing methods herein, priming can be done through random priming or primase-based initiation and then polymerase extension. Random primers can have sample barcode tails or adapter tails or barcoded adapter tails. Affinity molecules can be biotin or other suitable affinity tags, such as polyadenylation. Solid substrates can be magnetic beads, streptavidin coated plates, other suitable substrates. A solid substrate can be in a column, in a microfluidic device, in a pipet tip, or other suitable substrate surface.

In further embodiments of targeted sequencing methods herein, any suitable method that can create a double stranded, substrate bound conversion of all molecules in a sample that then can be separated physically through RNA guided endonuclease cleavage of target sequences is contemplated.

In additional embodiments of targeted sequencing methods herein, RNA guided endonucleases are used to target RNA/DNA duplex, DNA/DNA duplex, including with PAM sequences or without PAM sequences (utilizing CRISPER/Cas9 enzymes that do not need PAM sequences).

In further embodiments of targeted sequencing methods herein, single stranded DNA or RNA is captured on a bead and hybridized to a targeted oligonucleotide (makes target site only double stranded) to improve specificity.

Applications of targeted sequencing methods herein include but are not limited to full genome sequencing of known viral genomes, such as coronaviruses, respiratory viruses. Total RNA can be prepped and captured with Riptide^{®} libraries (e.g., a library construction technology comprising i) annealing an oligonucleotide comprising a first random primer and a barcoded adapter to a nucleic acid, ii) extending the first random primer and terminating the extension to generate an extension product, iii) annealing a second random primer with an adaptor to the extension product and generate a double-stranded extension product using the second random primer). Guide RNAs can be designed for every 500bp, 1kb, 3kb, or more and cleave off the viral cDNA sequences, which are separated and sequenced. In some cases, this enriches for the viral sequences from a complex background of total RNA from a sample. This can be done in high throughput setting or field deployable Oxford nanopore sequencing.

Additional applications of targeted sequencing include sequencing of ACMG gene list, such as BRCA genes. In additional embodiments, targeted sequencing includes carrier screening panel (all known recessive disorders).

In some cases, CRISPR cleavage is allele specific. For example, a specific allele can be clipped off and phased information obtained on a targeted locus such as HLA. Alternatively, low frequency cancer mutations can be targeted. Methods herein can also be used to target low abundance nuclear transcripts.

In another example of targeted sequencing methods herein, standard Riptide^{®} libraries (e.g., a library construction technology comprising i) annealing an oligonucleotide comprising a first random primer and a barcoded adapter to a nucleic acid, ii) extending the first random primer and terminating the extension to generate an extension product, iii) annealing a second random primer with an adaptor to the extension product and generate a double-stranded extension product using the second random primer) are generated for low pass sequencing and genotyping. The original libraries can be used to clip off the SNPs that were of interest in the study and in order to get high coverage confirmation of those variants in each sample.

One example of a workflow of the methods herein is as follows. Current method involving adapter ligation. Perform standard A reaction (primer extension reaction) with barcoded random primer without Illumina adapter. Capture primer-extended molecules with streptavidin magnetic beads. Wash beads three times to remove non-specifically bound DNA molecules. Perform primer extension reaction with barcoded random primer without Illumina adapter to create complementary strand (standard B reaction). Wash beads three times to remove non-specifically bound DNA molecules. Resuspend beads in 1X Cas9 buffer. Add pre-complexed Cas9:sgRNA to the streptavidin beads (sgRNA pool contains guide RNAs targeting all loci of interest). Incubate at 37 C for 30 - 60 mins for Cas9-mediated site-specific cleavage to occur. Clipped molecules will no longer be bound to beads. Place tube on magnetic stand. Transfer supernatant to new tube. Place tube back on magnetic stand. Transfer supernatant to new tube (this is to get rid of residual beads). Perform standard end-repair, phosphorylation and A-tailing reaction using reagents from the NEBNext Ultra II DNA Library Prep Kit (using the NEB protocol). Perform Illumina adapter ligation using reagents from the NEBNext Ultra II DNA Library Prep Kit (using the NEB protocol). Perform 0.9X Ampure bead cleanup or other bead-based size selection (using the NEB protocol). Perform PCR amplification with full length Illumina P5 and P7 primers (using the NEB protocol). Perform final Ampure bead cleanup (using the NEB protocol).

Another example of a workflow to improve the protocol by reducing off-target events and enriching for A primer barcode-containing molecules is as follows. Perform standard A reaction (primer extension reaction) with a barcoded random primer with partial P5 Illumina adapter and 5' amine group (to block 5' phosphorylation of DNA). Capture primer-extended molecules with streptavidin magnetic beads. Wash beads three times to remove non-specifically bound DNA molecules. Perform primer extension reaction (standard B reaction) with barcoded random primer without Illumina adapter but with 5' amine to create complementary strand. The complementary strand will be blocked for phosphorylation at the 5' end. Wash the beads twice to remove non-specifically bound DNA molecules. Block available 3' ends of the DNA molecules by treating with Terminal Transferase enzyme and dideoxynucleotides. Wash the beads twice more. Resuspend beads in 1X Cas9 buffer. Add pre-complexed Cas9:sgRNA to the streptavidin beads (sgRNA pool contains guide RNAs targeting all loci of interest). Incubate at 37 C for 30 - 60 mins for Cas9-mediated site-specific cleavage to occur. Place tube on magnetic stand. Transfer supernatant to new tube. Place tube back on magnetic stand. Pipette up supernatant again and transfer to a new tube (this is to get rid of residual beads). Perform standard end-repair, phosphorylation and A-tailing reaction using reagents from the NEBNext Ultra II DNA Library Prep Kit (using NEB or other protocol). Perform Illumina adapter ligation with P7 adapter only. Adapter will not ligate to the A primer end of the molecules or to any B primer ends present. Perform 0.9X Ampure bead cleanup or other bead-based size selection (using the NEB protocol). Perform PCR amplification with full length Illumina P5 and P7 primers. Perform final Ampure bead cleanup.

A further example of a workflow includes performing the normal RipTide library prep steps until Cas9 cleavage (steps 1 - 5 in the previous protocol, except with an A primer that carries a partial P5 Illumina adapter and a B primer without any Illumina adapter sequence), perform Cas9 cleavage, then perform transposition of cleaved products with P7 adapter-transposable ends and PCR amplify the P5-P7 products using full length Illumina P5 and P7 primers. There is no need for adapter ligation in this protocol. Transposition-based protocol is detailed below: Perform standard A reaction (primer extension reaction) with barcoded random primer carrying partial Illumina P5 adapter. Capture primer-extended molecules with streptavidin magnetic beads. Wash beads three times to remove non-specifically bound DNA molecules. Perform primer extension reaction with barcoded random primer without Illumina adapter to create complementary strand (standard B reaction). Wash beads three times to remove non-specifically bound DNA molecules. Resuspend beads in 1X Cas9 buffer. Add pre-complexed Cas9:sgRNA to the streptavidin beads (sgRNA pool contains guide RNAs targeting all loci of interest). Incubate at 37 C for 30 - 60 mins for Cas9-mediated site-specific cleavage to occur. Clipped molecules will no longer be bound to beads. Place tube on magnetic stand. Transfer supernatant to new tube. Place tube back on magnetic stand. Transfer supernatant to a new tube (this is to get rid of residual beads). Perform transposition with Nextera-like system. Illumina P7 adapters (no need for P5 adapters) will be inserted in CRISPR-cleaved molecules. Perform Ampure bead size selection (may or may not be required). Perform PCR amplification with full length Illumina P5 and P7 primers to amplify P5-P7 molecules. Perform final Ampure bead cleanup.

An alternate workflow includes performing target-specific transposition by employing a fusion of dCas9 to a transposase that would insert transposon ends with Illumina adapters at target sites of interest. There would be no need for adapter ligation after transposition nor would there be a need for separate Cas9 digestion and transposition events.

Another example workflow includes Cas12a (Cpf1), which cleaves dsDNA to create staggered cuts with 5' overhangs is as follows. Perform standard A reaction (primer extension reaction) with a barcoded random primer with partial P5 Illumina adapter (may include 5' amine group to block ligation). Capture primer-extended molecules with streptavidin magnetic beads. Wash beads three times to remove non-specifically bound DNA molecules. Perform primer extension reaction (standard B reaction) with barcoded random primer without Illumina adapter to create complementary strand. (primer may include 5' amine group to block ligation). Wash the beads three times to remove non-specifically bound DNA molecules. Resuspend beads in 1X Cas12a buffer. Add pre-complexed Cas 12a:gRNA to the streptavidin beads (gRNA pool contains guide RNAs targeting all loci of interest). Incubate at 37 C for Cas12a-mediated site-specific cleavage to occur. Clipped molecules will no longer be bound to beads. Place tube on magnetic stand. Transfer supernatant to new tube. Place tube back on magnetic stand. Transfer supernatant to new tube (this is to get rid of residual beads). Add Illumina P7 adapters with 3' single nucleotide overhangs (a mix of A, C, G and T overhangs). Treat with T4 DNA polymerase to fill in gap, then add T4 DNA ligase to seal the nicks. Perform 0.9X Ampure bead cleanup or other bead-based size selection (using the NEB protocol). Perform PCR amplification with full length Illumina P5 and P7 primers. Perform final Ampure bead cleanup

### Further embodiments

Aspects of the current disclosure describe methods of the invention and compositions not forming part of the invention for generating a targeted population of non-identical, tagged nucleic acid molecules each comprising a subset of sequence from a target nucleic acid sequence. The target nucleic acid sample may be obtained from any biological or environmental source, including plant, animal (including human), bacteria, fungi, or algae. Any suitable biological sample is used for the target nucleic acid. Convenient suitable samples include whole blood, tissue, semen, saliva, tears, urine, fecal material, sweat, buccal, skin, and hair. In some embodiments, the target nucleic acid is obtained from 50-500 cells. In some embodiments, the target nucleic acid is obtained from 50-400, 50-350, 50-300, 100-300, 150-300, 200-300, or 200-250 cells.

In an embodiment, the targeted sequencing method may comprise obtaining a first nucleic acid molecule comprising a first molecular tag sequence and a first target sequence having a first length from a target nucleic acid sample. The first nucleic acid molecule may be of varying length. In some embodiments, the length of the first nucleic acid molecule corresponds to the optimum length for a specific sequencing platform. Optimum lengths for specific sequencing platforms may include up to 400 nucleotide bases for ion semiconductor (*e.g.*, ION TORRENT, Life Technologies, Carlsbad, CA), 700 nucleotide bases for pyrosequencing (*e.g.*, GS JUNIOR+, 454 Life Sciences, Branford, CT), and 50 to 300 nucleotide bases for sequencing by synthesis (SBS) (*e.g.,* MISEQ, Illumina, San Diego, CA). In some embodiments, the first nucleic acid molecule may be 50-1000, 100-1000, 200-1000, 300-1000, 300-900, 300-800, 300-700, 300-600, 300-500, or 400-500 nucleotide bases. In some embodiments, the first nucleic acid molecule may be 50, 62.5, 125, 250, 500, or 1000 nucleotide bases.

In some embodiments, the first nucleic acid molecule comprises a molecular ligand. In some embodiments, this molecular ligand comprises biotin or any biotin derivatives or analogs.

In some embodiments, the molecular tag sequence may be 6, 7, 8, 9, or 10 nucleotide bases long. In some embodiments, the molecular tag is 8 nucleotide bases long. In an embodiment, the molecular tag comprises a random nucleotide sequence. In some embodiments, the random nucleotide sequence is synthesized in a semi-random fashion to account for variable content in a target nucleic acid sample. The random nucleotide sequence may be selected to reflect representative "randomness" ordered against the windows of guanine-cytosine (GC) content in the genome from 1% to 100% GC and synthesized and pooled in ratios relative to the content of the genome at each GC%.

In some embodiments, the first nucleic acid molecule may be obtained through contacting a first primer comprising a first random oligonucleotide sequence to a target nucleic acid sample. In some embodiments, contacting a first primer comprises annealing a first primer to a nucleic acid of said target nucleic acid sample. Annealing may result in complete hybridization or incomplete hybridization. In a further embodiment, a second nucleic acid is generated through contacting a second primer comprising a second random oligonucleotide sequence to a first nucleic acid molecule. This method may comprise annealing an oligonucleotide comprising a second molecular tag sequence to a first nucleic acid molecule and extending the oligonucleotide to obtain a first double-stranded nucleic acid molecule comprising a first molecular tag sequence, a first target sequence having a first length, and a second molecular tag sequence.

The targeted sequencing methods described herein may further comprise obtaining a second double-stranded nucleic acid molecule comprising a third molecular tag sequence, a second target sequence having a second length, and a fourth molecular tag sequence, and discarding the second double-stranded nucleic acid molecule if the third molecular tag sequence is identical to the first molecular tag sequence, the fourth molecular tag sequence is identical to the second molecular tag sequence, the second target sequence is identical to the first target sequence, and the second target sequence length is identical to the first target sequence length. In some embodiments, the second double-stranded molecule may be retained if the third molecular tag sequence is different from the first molecular tag sequence, the fourth molecular tag sequence is different from the second molecular tag sequence, the second target sequence is different from the first target sequence; or the second target sequence length is different from the first target sequence length, the result being generating a population of non-identical, tagged nucleic acid molecules each comprising a subset of sequence from a target nucleic acid sample

In some embodiments, the first nucleic acid comprises an adapter sequence positioned 5' to said first random oligonucleotide sequence. In some embodiments, this adapter sequence is added to facilitate amplification and/or sequencing for a specific sequencing platform. Sequencing platforms include ion semiconductor (*e.g.,* ION TORRENT, Life Technologies, Carlsbad, CA), pyrosequencing *(e.g.,* GS JUNIOR+, 454 Life Sciences, Branford, CT), and sequencing by synthesis (SBS) *(e.g.,* MISEQ, Illumina, San Diego, CA). Exemplary adapter sequences include SEQ ID NOs: 1 and 2.

In some cases, targeted sequencing library molecules are circularized prior to sequencing. Library molecule circularization is effected, for example, by providing a 'bridge oligo' or 'splint oligo' comprising sequence reverse-complementary to adapter sequences SEQ ID NO: 1 and SEQ ID NO: 2, or other adapter sequences, such that the 5' end and 3' end of a single-stranded library product molecule are simultaneously bound by the bridge oligo. In some cases the bridge oligo holds the 5' and 3' ends of the single-stranded library molecule in proximity through base-pairing hydrogen bond interactions, such that the 5' and 3' ends of a molecule may be joined upon addition of a ligase to form a circularized library molecule. Molecules may be circularized through any number of molecular techniques, such as ligation, cre-lox based fusion, nick-repair-based techniques or otherwise to form a single circular molecule. In some cases, libraries are then treated with exonuclease to remove bridge oligos.

Circularized molecules are then sequenced through one of a number of sequencing techniques known in the art, such as rolling circle amplification/sequencing to obtain sequence information.

In some cases, the first nucleic acid and the first primer may be contacted to a nucleic acid polymerase and a nucleotide triphosphate. Nucleic acid polymerases include DNA polymerases from the families A, B, C, D, X, Y, and RT. In some embodiments, the nucleic acid polymerase has strand displacement activity. In some embodiments, the nucleic acid polymerase lacks strand displacement activity. Nucleotide triphosphates can include deoxyribonucleoside triphosphates such as dATP, dCTP, dITP, dUTP, dGTP, and dTTP, and dideoxyribonucleoside triphosphates (ddNTPs) such as ddATP, ddCTP, ddGTP, ddITP, and ddTTP. In some embodiments, the nucleotide triphosphate is selected by the nucleic acid polymerase from a pool comprising deoxynucleotide triphosphates and dideoxynucleotide triphosphates. In some embodiments, this pool may comprise dideoxynucleotide triphosphates in an amount ranging from 0.01% - 5.0%, 0.01% - 4.0%, 0.01% - 3.0%, 0.01% - 2.0%, 0.02% - 2.0%, 0.03% - 2.0%, 0.04% - 2.0%, 0.05% - 2.0%, 0.06% - 2.0%, 0.07% - 2.0%, 0.08% - 2.0%, 0.09% - 2.0%, or 0.1% - 2.0%. In some embodiments, the pool may comprise dideoxynucleotide triphosphates in an amount of 0.05, 0.1%, 0.2%, 0.4%, 0.8%, or 1.0%. In some embodiments, the nucleotide triphosphate is selected by the nucleic acid polymerase from a pool comprising dATP, dCTP, dGTP, and dTTP, with one of the four deoxynucleotide triphosphates at a significantly lower concentration than the other three, or two of the four deoxynucleotide triphosphates at a significantly lower concentration than the other two. In some cases, the nucleotide triphosphate is selected by the nucleic acid polymerase from a pool of deoxynucleotide triphosphates and modified nucleotides, such as 2,6 Diaminopurine and 2-thiothymidine (or uracil, without a methyl group at 5 position). In some cases the modified nucleotides comprise a 'semi-compatible' nucleotide base pair. In some cases semi-compatible nucleotide base pairs comprise modified nucleotides selected such that they are able to base pair with a naturally occurring nucleotide base or bases that pair with their naturally occurring relative, but are unable to base pair with an analogue of their naturally occurring base pair partner. For example, the Adenine analogue 2,6-diaminopurine is able to base pair with Thymidine, and the Thymidine analogue 2-thiothymidine is able to base pair with Adenine, but the semi-compatible pair of 2,6-diaminopurine and 2-thiothymidine cannot base pair with one another. This, the Adenine analogue 2,6-diaminopurine and the Thymidine analogue 2-thiothymidine constitute a semi-compatible base pair. A composition comprising the nucleotide triphosphates dGTP and dCTP (a complementary or natural pair), and the semi-complementary pair deoxy-2,6-diaminopurineTP and deoxy-2-thiothymidineTP, thus, supports extension from a 3'OH position of template-directed nucleic acid synthesis.

Other modified base pairings are contemplated, such as alternative A:T pairs and alternative G:C pairs.

A benefit of such semi-compatible modified bases is that a nucleic acid template incorporating these modified bases cannot serve as a template for synthesis if the dNTP pool from which nucleic acids are drawn includes a sufficient concentration of these bases. Thus, nucleic acids incorporating these bases are confidently templated by an original nucleic acid sample rather than being templated by other synthesized nucleic acids. This characteristic allows the synthesis of multiple copies of a sample nucleic acid without the risk that a base incorporation mismatch error early in the nucleic acid synthesis reaction will be propagated in later templates. However, by replacing the dNTP pool with a pool consisting of or comprising naturally occurring dNTP of the type of base for which the analogue is a replacement, nucleic acids comprising all four naturally occurring bases is generated from templates incorporating base pair analogues.

In some cases, at least one of the modified nucleotides is labeled. In some cases at least one of the modified nucleotides is digoxigenin(DIG)-, biotin-, fluorescein-, or tetramethylrhodamine-labeled. In some cases, the template is fragmented into fragments of a specific length prior to contacting the first nucleic acid and the first primer. In some cases one or more nucleotide analogs are used, such as nucleotide analogs that are sensitive to endonuclease treatment in combination with an endonuclease to achieve chain termination. In some cases chain termination is achieved through manipulation of dNTP concentration

In an embodiment, a pool comprising deoxynucleotide triphosphates and dideoxynucleotide triphosphates comprises at least one dideoxynucleotide triphosphate bound to a molecular ligand. In some embodiments, this molecular ligand comprises biotin. In some embodiments, the methods comprise contacting a molecule comprising an oligonucleotide comprising a second molecular tag sequence annealed to said first nucleic acid molecule to a ligand binding agent. In some embodiments, this ligand binding agent is avidin or streptavidin. In some cases, the ligand binding agent is a high-affinity antibody to DIG, biotin, fluorescein, or tetramethylrhodamine.

In some embodiments, at least one of the nucleic acids described herein is a deoxyribonucleic acid. In a further embodiment, a deoxyribonucleic acid is fragmented into fragments greater than 10 kilobases. Fragmentation may be accomplished in a number of ways, including mechanical shearing or enzymatic digestion. In some embodiments, at least one of the nucleic acids described herein is a ribonucleic acid. In some embodiments, a target nucleic acid sample is ribonucleic acid. In a further embodiment, a first nucleic acid molecule is a complementary deoxyribonucleic acid (cDNA) molecule generated from a ribonucleic acid. In some embodiments, the nucleic acid polymerase that generated the cDNA is an RNA-dependent DNA polymerase. In some embodiments, the cDNA is generated through contacting a first primer comprising an oligo(dT) sequence to a target nucleic acid sample.

In a further embodiment, all sequences from a given contig having the same molecular tag are assigned to a specific homologous chromosome.

Also described herein but not forming part of the invention are targeted sequencing compositions comprising a first nucleic acid molecule comprising a first molecular tag sequence and a first target sequence having a first length, and an oligonucleotide comprising a second molecular tag sequence. The first nucleic acid molecule may comprise a 3' deoxynucleotide. The 3' deoxynucleotide may be a dideoxynucleotide. The first nucleic acid may comprise an adapter sequence positioned 5' to the first molecular tag sequence. This adapter sequence may be added to facilitate amplification and/or sequencing for a specific sequencing platform, such as ion semiconductor (e.g., ION TORRENT, Life Technologies, Carlsbad, CA), pyrosequencing (e.g., GS JUNIOR+, 454 Life Sciences, Branford, CT), or sequencing by synthesis (SBS) (e.g., MISEQ, Illumina, San Diego, CA). Exemplary adapter sequences include 5' AAT GAT ACG GCG ACC ACC GA 3' (SEQ ID NO: 1), and 5' CAA GCA GAA GAC GGC ATA CGA GAT 3' (SEQ ID NO: 2). Adapters compatible with Illumina, 454, Ion Torrent and other known sequencing technologies are contemplated herein.

The targeted sequencing composition may comprise a first nucleic acid molecule comprising a molecular ligand. This molecular ligand may comprise biotin. The composition may comprise a ligand binding agent. This ligand binding agent may be avidin or streptavidin. The compositions described herein may also comprise a ligand-ligand binding agent wash buffer. The compositions described herein may comprise a biotin wash buffer.

The targeted sequencing compositions described herein but not forming part of the invention may also comprise unincorporated nucleotides. The unincorporated nucleotides may be unincorporated deoxynucleotides. The unincorporated nucleotides may be dideoxynucleotides.

The compositions described herein but not forming part of the invention may comprise a first nucleic acid molecule hybridized to an oligonucleotide comprising a second molecular tag sequence. The first nucleic acid molecule may be completely hybridized to the second molecular tag sequence of the oligonucleotide, or the first nucleic acid molecule may be incompletely hybridized to the second molecular tag sequence of the oligonucleotide.

Further described herein but not forming part of the invention are targeted sequencing compositions comprising a population of nucleic acid molecules, wherein each molecule independently comprises a first strand comprising a first adapter sequence, a molecular tag sequence, and an independent target sequence, and wherein each independent target sequence comprises a subset of a sample nucleic acid sequence, and wherein at least a first molecule of the population comprises an independent target sequence comprising a first subset of the sample nucleic acid sequence, and wherein at least a second molecule of the population comprises an independent target sequence that comprises a second subset of the sample nucleic acid sequence. The adapter of each first strand of the population may be identical. The molecular tag sequence of each molecule of the population may comprise at least six nucleotide bases. A first member of the population and a second member of the population may comprise non-identical molecular tag sequences. Each first strand may comprise a 3'-doexynucleotide base at its 3' end. Each first strand may comprise a molecular ligand at its 5' end or each first strand may comprise a molecular ligand attached at a non-terminal position. Additionally, each first strand may comprise a molecular ligand at its 3' end. The molecular ligand may be biotin.

The compositions described herein but not forming part of the invention may comprise a population of nucleic acid molecules, wherein each molecule of the population comprises a second strand comprising a second adapter sequence and a second molecular tag sequence. The second strand of at least one molecule of the population may be annealed to a first strand via at least partial base pairing of a second molecular tag sequence of the second strand to the independent target sequence of the first strand. The adapter of each second strand of the population may be identical. At least one molecule of the population may be bound to a molecular ligand binder. The molecular ligand binder may comprise avidin or streptavidin.

The targeted sequencing compositions described herein but not forming part of the invention may also comprise unincorporated nucleic acid triphosphates. The compositions described herein but not forming part of the invention may comprise molecular ligand binder wash buffer, and/or polymerase extension buffer, and/or nucleic acid polymerase. The nucleic acid polymerase may possess nucleic acid helicase activity. The compositions described herein but not forming part of the invention may comprise nucleic acid polymerase possessing nucleic acid strand displacement activity. The compositions described herein but not forming part of the invention may comprise the sequences compatible with Illumina, Ion torrent or 454 sequencing technology. The compositions described herein may comprise the sequences recited in SEQ ID NO: 1 and SEQ ID NO: 2.

Targeted sequence information obtained herein is used in some cases to quantify nucleic acid accumulation levels. A library is generated and sequenced as disclosed herein. Duplicate reads are excluded so that only uniquely tagged reads are included. Unique read sequences are mapped to a genomic sequence or to a cDNA library or transcriptome sequence, such as a transcriptome for a given cell type or treatment or a larger transcriptome set up to and including an entire transcriptome set for an organism. The number of unique library sequence reads mapping to a target region is counted and is used to represent the abundance of that sequence in the sample. In some embodiments uniquely tagged sequence reads each map to a single site in the sample sequence. In some cases, uniquely tagged sequence reads map to a plurality of sites throughout a genome, such as transposon insertion sites or repetitive element sites. Accordingly, in some cases the number of library molecules mapping to a transcriptome 'locus' or transcript corresponds to the level of accumulation of that transcript in the sample from which the library is generated. The number of library molecules mapping to a repetitive element, relative to the number of library molecules that map to a given unique region of the genome, is indicative of the relative abundance of the repetitive element in the sample. Thus, disclosed herein is a method of quantifying the relative abundance of a nucleic acid molecule sequence in a sample comprising the steps of generating a sequence library comprising uniquely tagged library fragments and mapping the nucleic acid molecule sequence onto the library, such as the frequency of occurrence of the nucleic acid molecule sequence in the library corresponds to the abundance of the nucleic acid molecule sequence in the sample from which the library is generated. In some cases the frequency of occurrence of the nucleic acid molecule sequence in the library is assessed relative to the frequency of occurrence of a second nucleic acid molecule sequence in the library, said second nucleic acid sequence corresponding to a locus or transcript of known abundance in a transcriptome or known copy number per genome of a genomic sample.

Methods of preparing nucleic acids in a sample for targeted sequencing using any of the compositions are described herein. In some embodiments, the samples is obtained from a cell, a tissue, or a partial of an organism. Non-limiting examples of organisms can include, human, plants, bacteria, virus, protozoans, eukaryotes, and prokaryotes. As an illustrating example, the sample is a human genome comprising human genomic nucleic acids. The sample is used to prepare a nucleic acid library. The library is sequenced.

Preparation of nucleic acid library for targeted sequencing is achieved using methods as described herein or methods known in the art. In some embodiments, the nucleic acids are obtained from a human genome. The human genome nucleic acids is amplified in a reaction mixture X. In some embodiments, the reaction mixture X can comprise DNA, at least one primer, a buffer, a deoxynucleotide mixture, an enzyme, and nuclease-free water. The reaction mixture X is prepared in an Eppendorf tube. Preferably, the reaction mixture X is prepared in an Eppendorf DNA LoBind microcentrifuge tube. In some cases, the DNA is a human DNA. The final concentration of DNA in the reaction mixture X is about 0.1 ng, 0.2 ng, 0.3 ng, 0.4 ng, 0.5 ng, 0.6 ng, 0.7 ng, 0.8 ng, 0.9 ng, 1.0 ng, 1.2 ng, 1.4 ng, 1.5 ng, 1.8 ng, 2.0 ng, or more. The final concentration of DNA in the reaction mixture X is about 0.1 ng, 0.2 ng, 0.3 ng, 0.4 ng, 0.5 ng, 0.6 ng, 0.7 ng, 0.8 ng, 0.9 ng, 1.0 ng, 1.2 ng, 1.4 ng, 1.5 ng, 1.8 ng, 2.0 ng, or less. The final concentration of DNA in the reaction mixture X is between about 0.1 to about 2.0 ng, between about 0.2 ng to about 1.2 ng, between about 0.5 ng to about 0.8 ng, or between about 1.0 ng to about 1.5 ng.

In some cases, the reaction mixture X comprises only one primer, for example, Primer A. The final concentration of Primer A in the total reaction mixture is about 10 µM, 20 µM, 30 µM, 40 µM, about 50 µM, about 100 µM, about 150 µM, about 200 µM, or more. The final concentration of Primer A in the total reaction mixture X is about 10 µM, 20 µM, 30 µM, 40 µM, about 50 µM, about 100 µM, about 150 µM, about 200 µM, or less. The final concentration of Primer A in the total reaction mixture X is between about 10 µM to about 200 µM, between about 30 µM to about 80 µM, between about 50 µM to about 100 µM, or between about 40 µM, to about 150 µM.

In some cases, the reaction mixture X comprises a buffer such as a Thermo Sequenase Buffer. Typically, the final concentration of buffer in the reaction mixture X is about 10% of the original concentration of the buffer. For example, depending on the final volume of the reaction mixture X, the amount of buffer to be added is less than, more than or about 1 µl, about 2 µl, about 2.5 µl, about 3 µl, about 4 µl, about 5 µl, about 10 µl.

In some cases, the reaction mixture X comprises a plurality of deoxynucleotides. The deoxynucleotides are one or more of dATP, dTTP, dGTP, dCTP, ddATP, ddTTP, ddGTP and ddCTP. The final concentration of deoxynucleotides in the reaction mixture X is about 0.1 µM, about 0.2 µM, about 0.3 µM, about 0.4 µM, about 0.5 µM, about 0.6 µM, about 0.7 µM, about 0.8 µM, about 0.9 µM, about 1.0 µM, about 1.2 µM, about 1.5 µM, about 1.8 µM, about 2.0 µM, or more. The final concentration of deoxynucleotides in the reaction mixture X is about 0.1 µM, about 0.2 µM, about 0.3 µM, about 0.4 µM, about 0.5 µM, about 0.6 µM, about 0.7 µM, about 0.8 µM, about 0.9 µM, about 1.0 µM, about 1.2 µM, about 1.5 µM, about 1.8 µM, about 2.0 µM, or less.

In some cases, the reaction mixture X comprises an enzyme such as a polymerase. For example, the enzyme is a Thermo Sequenase in some cases. The final concentration of the polymerase is about 0.01 µM, about 0.1 µM, about 0.2 µM, about 0.3 µM, about 0.4 µM, about 0.5 µM, about 0.6 µM, about 0.7 µM, about 0.8 µM, about 0.9 µM, about 1.0 µM, about 1.2 µM, about 1.5 µM, about 1.8 µM, about 2.0 µM, or more. The final concentration of the polymerase is about 0.01 µM, about 0.1 µM, about 0.2 µM, about 0.3 µM, about 0.4 µM, about 0.5 µM, about 0.6 µM, about 0.7 µM, about 0.8 µM, about 0.9 µM, about 1.0 µM, about 1.2 µM, about 1.5 µM, about 1.8 µM, about 2.0 µM, or less. The final concentration of the polymerase is between to about 2.0 µM, between about 0.1 µM to about 1.0 µM, between about 0.5 µM to about 1.5 µM, or between about 0.8 µM to about 1.8 µM.

Typically, a volume of nuclease-free water is added to the reaction mixture X to achieve a desired final volume. The final volume of the reaction mixture is about 10 µl, about 20 µl, about 25 µl, about 30 µl, about 40 µl, about 50 µl, or about 100 µl. Depending on the final volume of reaction mixture X, the amount of nuclease-free water is about 0.1 µl, about 0.5 µl, about 0.8 µl, about 1.0 µl, about 2 µl, about 5 µl, about 10 µl, about 15 µl, about 20 µl, about 25 µl, about 30 µl, about 40 µl, about 50 µl, about 80 µl, about 90 µl, about 95 µl, or more. The amount of nuclease-free water is about 0.1 µl, about 0.5 µl, about 0.8 µl, about 1.0 µl, about 2 µl, about 5 µl, about 10 µl, about 15 µl, about 20 µl, about 25 µl, about 30 µl, about 40 µl, about 50 µl, about 80 µl, about 90 µl, about 95 µl, or less. The amount of nuclease-free water is between about 0.1 µl to about 95 µl, between about 1.0 µl to about 10 µl, between about 5 µl to about 50 µl, or between about 20 µl to about 80 µl.

In general, the reaction mixture X is incubated at a temperature (Tm) for a period of time long enough to denature the DNA. The Tm is about 80 °C, about 85 °C, about 90 °C , about 91 °C, about 92 °C, about 93 °C, about 94 °C, about 95 °C, about 96 °C, about 97 °C, about 98 °C, about 99 °C, or more. The reaction mixture X is incubated at Tm for more than, less than, or about 5 seconds, about 10 seconds, about 15 seconds, about 20 seconds, about 30 seconds, about 1 minute, about 2 minutes, about 3 minutes, about 4 minute, about 5 minutes, about 6 minutes, about 7 minutes, about 8 minutes, about 9 minutes, about 10 minutes. For example, the reaction mixture X is incubated at 95 °C for about 3 minutes. After denaturing, the temperature of the reaction mixture X is lowered by placing the tube on ice. For example, the tube is placed on ice for more than, less than, or about 5 seconds, about 10 seconds, about 15 seconds, about 20 seconds, about 30 seconds, about 5 seconds, about 10 seconds, about 15 seconds, about 20 seconds, about 30 seconds, about 1 minute, about 2 minutes, about 3 minutes, about 4 minute, about 5 minutes, about 6 minutes, about 7 minutes, about 8 minutes, about 9 minutes, about 10 minutes. Preferably, the polymerase, for example, Thermo Sequenase, is added to the reaction, and mixed gently. In general, the reaction mixture X is transferred to a thermal cycler, and proceed with a problem on the instrument described herein.

The thermal cycler performs a program comprising (1) maintaining the temperature at about a low temperature for a period of time, (2) increasing the temperature to a DNA annealing temperature, (3) maintaining at the annealing temperature for a period of time, (4) increasing the temperature to a denature temperature for a period of time, repeating (1) to (4) for at least 9 times, and hold at 8 °C, 4 °C, or lower, or frozen at -20 °C for storage. The low temperature of (1) is maintained at about 10 °C , about 12 °C, about 14 °C, about 16 °C, about 18 °C, or about 20 °C. The low temperature of (1) is maintained for about 5 seconds, about 10 seconds, about 15 seconds, about 20 seconds, about 30 seconds, about 1 minute, about 2 minutes, about 3 minutes, about 4 minute, about 5 minutes, about 6 minutes, about 7 minutes, about 8 minutes, about 9 minutes, about 10 minutes, about 15 minutes, or about 20 minutes. As an alternative, the thermal cycler can maintain the temperature at about 16 °C for about 3 minutes. In some embodiments, the temperature from (1) to (2) is increased slowly, such that the temperature is ramp out by a small increment of temperature at about 0.1 °C/second. The temperature of (2) is about 45 °C, about 50 °C, about 55 °C, about 60 °C, about 65 °C, about 68 °C, about 70 °C, or more. In some cases, the temperature of (2) is slowly ramped up to about 60 °C by 0.1 °C/second. In some cases, the temperature of (2) is the same as the temperature of (3). In some cases, the temperature of (2) is further increased to reach the temperature of (3). The temperature of (3) is maintained for about 5 seconds, about 10 seconds, about 15 seconds, about 20 seconds, about 30 seconds, about 1 minute, about 2 minutes, about 3 minutes, about 4 minute, about 5 minutes, about 6 minutes, about 7 minutes, about 8 minutes, about 9 minutes, about 10 minutes, about 15 minutes, or about 20 minutes. In some embodiments, the temperature of (3) is maintained for about 10 minutes. As an example, the temperature of (4) is about 95 °C, and maintained for about 10 seconds, 20 seconds, 30 seconds, 45 seconds, 60 seconds, 1 minute, 2 minutes, or longer.

In some embodiments, all reaction components in the reaction mixture X, except the primer, are combined and loaded onto a relevant partitioning device. After the reaction tis partitioned and combined with barcoded primers, the reaction mixture is transferred to a thermal cycler, heat denatured at 95 °C for 2 minutes, and subsequently thermocycled according to the program described herein. In some embodiments, the product is temporarily stored at 4 °C or on ice, or frozen at -20 °C for long term storage. In some embodiments, shortly before continuing with the next step, the stored product is heated at about 98 °C for about 3 minutes, then transferred to temporarily store on ice.

In some embodiments, the DNA product of the reaction mixture X described above is captured with magnetic beads. This is achieved by preparing the Capture Beads prior to adding the product as described above. To begin with, the Capture Bead tube is shook thoroughly to resuspend the beads and transfer about 40 µl of the beads to a new 0.5 mL Eppendorf DNA LoBind tube. In some cases, the volume of beads is about 10 µl, about 20 µl, about 30 µl, about 50 µl, about 100 µl, or more. The tube is placed on a magnetic stand for about 0.5-1 minutes to allow the solution to clear up. The supernatant is pipetted and discarded. The tube is removed from the magnetic stand. A volume of about 200 µl of HS Buffer is added to the beads. The components are mixed gently by pipetting the sample up and down, before returning to the magnetic stand. The sample is kept on the magnetic stand for about 0.5-1 minutes to allow the solution to clear up. The supernatant is removed and discarded by gently pipetting it out of the tube. The tube is then removed from the magnetic stand and the beads are resuspended in 40 µl of HS Buffer. The tube is temporarily left on the laboratory bench at room temperature. The DNA product from the reaction mixture described above is added to be Capture Beads prepared as described herein, and incubated at room temperature for about 20 minutes. In some case, the sample comprising the DNA and Capture Beads is incubated at room temperature for about 10 minutes, about 15 minutes, about 20 minutes, about 30 minutes, or more. The DNA product and the Capture Beads is mixed by pipetting up and down for about 5 minutes, about 10 minutes, about 15 minutes, about 20 minutes, about 30 minutes, or more. The tube comprising the mixture of DNA product and Capture Beads is placed on the magnetic stand and wait for the solution to clear up. The supernatant is removed by carefully pipetting it out of the tube. The tube can then be removed from the magnetic stand and the beads is resuspended in 200 µl of Bead Wash Buffer, and returned to the magnetic stand for a period of time to allow the solution to clear up. The supernatant is discarded. The washing is repeated for at least 2 additional times, and the remaining liquid after the final wash is carefully removed.

The washed Capture Beads and DNA product described above is added to a mixture of reagents to generate a reaction mixture Y. The reagent can comprise a Sequenase buffer, a plurality of deoxynucleotides, at least one primer, an enzyme, and nuclease-Free water.

In some cases, the reaction mixture Y comprises only one primer, for example, Primer B. The final concentration of Primer A in the total reaction mixture Y is about 10 µM, 20 µM, 30 µM, 40 µM, about 50 µM, about 100 µM, about 150 µM, about 200 µM, or more. The final concentration of Primer B in the total reaction mixture Y is about 10 µM, 20 µM, 30 µM, 40 µM, about 50 µM, about 100 µM, about 150 µM, about 200 µM, or less. The final concentration of Primer B in the total reaction mixture Y is between about 10 µM to about 200 µM, between about 30 µM to about 80 µM, between about 50 µM to about 100 µM, or between about 40 µM, to about 150 µM.

In some cases, the reaction mixture Y comprises a Sequenase Buffer. Typically, the final concentration of buffer in the reaction mixture Y is about 10% of the original concentration of the buffer. In some cases, the final concentration of buffer in the reaction mixture Y is about 5%, about 10%, about 15%, about 20%, about 30% or less, of the original concentration of the buffer. For example, depending on the final volume of the reaction mixture Y, the amount of buffer to be added is less than, more than or about 1 µl, about 2 µl, about 2.5 µl, about 3 µl, about 4 µl, about 5 µl, about 10 µl.

In some cases, the reaction mixture Y comprises a plurality of deoxynucleotides. The deoxynucleotides is dATP, dTTP, dGTP, dCTP, ddATP, ddTTP, ddGTP and ddCTP. The final concentration of deoxynucleotides in the reaction mixture Y is about 0.1 µM, about 0.2 µM, about 0.3 µM, about 0.4 µM, about 0.5 µM, about 0.6 µM, about 0.7 µM, about 0.8 µM, about 0.9 µM, about 1.0 µM, about 1.2 µM, about 1.5 µM, about 1.8 µM, about 2.0 µM, or more. The final concentration of deoxynucleotides in the reaction mixture Y is about 0.1 µM, about 0.2 µM, about 0.3 µM, about 0.4 µM, about 0.5 µM, about 0.6 µM, about 0.7 µM, about 0.8 µM, about 0.9 µM, about 1.0 µM, about 1.2 µM, about 1.5 µM, about 1.8 µM, about 2.0 µM, or less.

In some cases, the reaction mixture Y comprises an enzyme. The enzyme is a polymerase. For example, the enzyme is a Sequenase. In some cases, the Sequenases comprises 1:1 ratio of Sequenase and Inorganic Pyrophosphatase. The final concentration of the polymerase is about 0.01 µM, about 0.1 µM, about 0.2 µM, about 0.3 µM, about 0.4 µM, about 0.5 µM, about 0.6 µM, about 0.7 µM, about 0.8 µM, about 0.9 µM, about 1.0 µM, about 1.2 µM, about 1.5 µM, about 1.8 µM, about 2.0 µM, or more. The final concentration of the polymerase is about 0.01 µM, about 0.1 µM, about 0.2 µM, about 0.3 µM, about 0.4 µM, about 0.5 µM, about 0.6 µM, about 0.7 µM, about 0.8 µM, about 0.9 µM, about 1.0 µM, about 1.2 µM, about 1.5 µM, about 1.8 µM, about 2.0 µM, or less. The final concentration of the polymerase is between to about 2.0 µM, between about 0.1 µM to about 1.0 µM, between about 0.5 µM to about 1.5 µM, or between about 0.8 µM to about 1.8 µM.

Typically, a volume of nuclease-free water is added to the reaction mixture to achieve a desired final volume. The final volume of the reaction mixture Y is about 10 µl, about 20 µl, about 25 µl, about 30 µl, about 40 µl, about 50 µl, or about 100 µl. Depending on the final volume of reaction mixture, the amount of nuclease-free water is about 0.1 µl, about 0.5 µl, about 0.8 µl, about 1.0 µl, about 2 µl, about 5 µl, about 10 µl, about 15 µl, about 20 µl, about 25 µl, about 30 µl, about 40 µl, about 50 µl, about 80 µl, about 90 µl, about 95 µl, or more. The amount of nuclease-free water is about 0.1 µl, about 0.5 µl, about 0.8 µl, about 1.0 µl, about 2 µl, about 5 µl, about 10 µl, about 15 µl, about 20 µl, about 25 µl, about 30 µl, about 40 µl, about 50 µl, about 80 µl, about 90 µl, about 95 µl, or less. The amount of nuclease-free water is between about 0.1 µl to about 95 µl, between about 1.0 µl to about 10 µl, between about 5 µl to about 50 µl, or between about 20 µl to about 80 µl.

In some embodiments, the reaction mixture Y is incubated for about 20 minutes at 24 °C. The mixture is incubated for a longer or a shorter time. For example, the reaction mixture Y is incubated for about 10 minutes, about 15 minutes, about 20 minutes, about 30 minutes, or more. The temperature is more than, less than, or about 18 °C, about 20 °C, about 25 °C, about 28 °C. preferably, the incubation is performed in a thermal cycler or heating block. The tube can then be placed on a magnetic stand for a period of time to allow the solution to clear up. The supernatant is removed and discarded. The tube is then removed from the magnetic sand and the beads are resuspended in about 200 µl of Bead Wash Buffer, before returning to the magnetic stand, left to sit until the solution clear up. The supernatant is carefully removed. The washing procedures is typically repeated for at least additional 2 times. The remaining liquid after the final wash is carefully removed.

In some embodiments, the reaction Y is added to a reaction mixture to generate reaction mixture Z. In general, the reaction Y is added to a reaction mixture Z in a PCR tube comprising a PCR Universal Primer I, a PCR Primer II with barcodes, a KAPA HiFi PCR Amplification Mix, and Nuclease-Free water.

In some cases, the final concentration of PCR Universal Primer I in the total reaction mixture Z' is about 10 µM, 20 µM, 30 µM, 40 µM, about 50 µM, about 100 µM, about 150 µM, about 200 µM, or more. The final concentration of PCR Universal Primer I in the total reaction mixture Z' is about 10 µM, 20 µM, 30 µM, 40 µM, about 50 µM, about 100 µM, about 150 µM, about 200 µM, or less. The final concentration of PCR Universal Primer I in the total reaction mixture Z' is between about 10 µM to about 200 µM, between about 30 µM to about 80 µM, between about 50 µM to about 100 µM, or between about 40 µM, to about 150 µM.

In some cases, the final concentration of PCR Primer II in the total reaction mixture Z' is about 10 µM, 20 µM, 30 µM, 40 µM, about 50 µM, about 100 µM, about 150 µM, about 200 µM, or more. The final concentration of PCR Primer II in the total reaction mixture Z' is about 10 µM, 20 µM, 30 µM, 40 µM, about 50 µM, about 100 µM, about 150 µM, about 200 µM, or less. The final concentration of PCR Primer II in the total reaction mixture Z' is between about 10 µM to about 200 µM, between about 30 µM to about 80 µM, between about 50 µM to about 100 µM, or between about 40 µM, to about 150 µM.

In some cases, the reaction mixture comprises a KAPA HiFi PCR Amplification Mix. Typically, the final concentration of KAPA HiFi PCR Amplification Mix in the reaction mixture Z' is about 10% of the original concentration of the mix. In some cases, the final concentration of KAPA HiFi PCR Amplification Mix in the reaction mixture Z' is about 5%, about 10%, about 15%, about 20%, about 30% or less, of the original concentration of the mix. For example, depending on the final volume of the reaction mixture Z', the amount of KAPA HiFi PCR Amplification Mix to be added is less than, more than or about 1 µl, about 2 µl, about 2.5 µl, about 3 µl, about 4 µl, about 5 µl, about 10 µl.

Typically, a volume of nuclease-free water is added to the reaction mixture Z' to achieve a desired final volume. The final volume of the reaction mixture Z' is about 10 µl, about 20 µl, about 25 µl, about 30 µl, about 40 µl, about 50 µl, or about 100 µl. Depending on the final volume of reaction mixture, the amount of nuclease-free water is about 0.1 µl, about 0.5 µl, about 0.8 µl, about 1.0 µl, about 2 µl, about 5 µl, about 10 µl, about 15 µl, about 20 µl, about 25 µl, about 30 µl, about 40 µl, about 50 µl, about 80 µl, about 90 µl, about 95 µl, or more. The amount of nuclease-free water is about 0.1 µl, about 0.5 µl, about 0.8 µl, about 1.0 µl, about 2 µl, about 5 µl, about 10 µl, about 15 µl, about 20 µl, about 25 µl, about 30 µl, about 40 µl, about 50 µl, about 80 µl, about 90 µl, about 95 µl, or less. The amount of nuclease-free water is between about 0.1 µl to about 95 µl, between about 1.0 µl to about 10 µl, between about 5 µl to about 50 µl, or between about 20 µl to about 80 µl.

The reaction mixture Z is placed in a thermal cycler to perform a polymerase chain reaction (PCR) and generate a product of XX. The PCR program comprises at least 1 cycle at about 98 °C for 2 minutes for denaturing the DNA, at least 15 cycles at about 98 °C for 20 seconds for denaturing, lower the temperature to about 60 °C for 30 seconds for annealing the primers, increase the temperature to about 72 °C for 30 seconds for extension, at least 1 cycle at about 72 °C for 5 minutes for final extension, and kept at 4 °C. In some cases, the DNA denature temperature is about 92 °C, about 95 °C, about 97 °C, or about 99 °C. In some cases, the primer annealing temperature is about 45 °C, about 50 °C, about 55 °C, about 60 °C, about 65 °C, or about 70 °C. In some cases, the extension temperature is about 65 °C, about 70 °C, about 72 °C, or about 75 °C.

The product XX is cleaned with AmpureXP Beads. In general, the PCR tube comprising product XX is placed on a magnetic stand, and kept still for the solution to clear up until the supernatant is removed by pipetting. The supernatant is transferred to a new 0.5 mL Eppendorf DNA LoBind tube. The PCR tube containing the Capture Beads is discarded. Typically, about 100 µl of AmpureXP Beads are added to the supernatant, and the mixture is mixed by pipetting up and down, before incubating at room temperature for about 10 minutes. In some cases, the incubation time is longer or shorter than 10 minutes, such as about 5 minutes, about 15 minutes, about 20 minutes, about 30 minutes, or more. The tube is placed on the magnetic stand to allow the solution to clear up. The supernatant is discarded. About 200 µl of 80% ethanol is added to the tube, and let sit for about 30 seconds, before removing and discarding the ethanol. It may not be necessary to remove the tube from the magnetic stand during this procedure. The tube is washed with 200 µl of 80% ethanol for at least additional 1 time. The cap of the tube is opened and allow the beads to air dry for about 10 - 15 minutes. About 20 µl to about 30 µl of 10mM Tric-HCl (pH7.8) is added to the beads. The resulting mixture is mixed by pipetting up and down, before allowing to sit at room temperature for about 2 minutes. The tube is placed on the magnetic stand to allow the solution to clear. The supernatant containing the eluted DNA is transferred to a new Eppendorf DNA LoBind tube. The product can then be used to generate a library, and is quantitated on an Agilent Bioanalyzer using a high sensitivity DNA chip prior to sequencing.

It is observed that in some embodiments, all steps of library preparation up to this point are performed in a single volume. In some cases the single volume is a single tube. In some cases the single volume is a single well in a plate. Optionally, after library generation, the DNA is size selected using either bead-based or agarose gel-based methods and that the library is quantitated on an Agilent Bioanalyzer using a high sensitivity DNA chip prior to sequencing.

### Endonuclease for targeted cleavage of nucleic acid

Targeted sequencing methods disclosed herein comprise targeting cleavage of the nucleic acid using a site-specific, targetable, and/or engineered nuclease or nuclease system. Such nucleases may create double-stranded break (DSBs) at desired locations in a genomic, cDNA or other nucleic acid molecule. In other examples, a nuclease may create a single strand break. In some cases, two nucleases are used, each of which generates a single strand break. Many cleavage enzymes consistent with the disclosure herein share a trait that they yield molecules having an end accessible for single stranded or double stranded exonuclease activity.

Endonucleases consistent with the disclosure herein variously include at least one selected from Clustered Regulatory Interspaced Short palindromic Repeat (CRISPR)/Cas system protein-gRNA complexes, Zinc Finger Nucleases (ZFN), and Transcription activator like effector nucleases. In some embodiments, the gRNAs are complementary to at least one site on the target nucleic acid. Other programmable, nucleic acid sequence specific endonucleases are also consistent with the disclosure herein.

Engineered nucleases such as zinc finger nucleases (ZFNs), Transcription Activator-Like Effector Nucleases (TALENs), engineered homing endonucleases, and RNA or DNA guided endonucleases, such as CRISPR/Cas such as Cas9 or CPF1, and/or Argonaute systems, are particularly appropriate to carry out some of the methods of the present disclosure. Additionally or alternatively, RNA targeting systems may be used, such as CRISPR/Cas systems including c2c2 nucleases.

Methods disclosed herein may comprise cleaving a target nucleic acid using CRISPR systems, such as a Type I, Type II, Type III, Type IV, Type V, or Type VI CRISPR system. CRISPR/Cas systems may be multi-protein systems or single effector protein systems. Multi-protein, or Class 1, CRISPR systems include Type I, Type III, and Type IV systems. Alternatively, Class 2 systems include a single effector molecule and include Type II, Type V, and Type VI.

CRISPR systems used in some targeted sequencing methods disclosed herein may comprise a single or multiple effector proteins. An effector protein may comprise one or multiple nuclease domains. An effector protein may target DNA or RNA, and the DNA or RNA may be single stranded or double stranded. CRISPR systems may comprise a single or multiple guiding RNAs. The gRNA may comprise a crRNA. The gRNA may comprise a chimeric RNA with crRNA and tracrRNA sequences. The gRNA may comprise a separate crRNA and tracrRNA. Target nucleic acid sequences may comprise a protospacer adjacent motif (PAM) or a protospacer flanking site (PFS). The PAM or PFS may be 3' or 5' of the target or protospacer site. Cleavage of a target sequence may generate blunt ends, 3' overhangs, or 5' overhangs.

A gRNA may comprise a spacer sequence. Spacer sequences may be complementary to target sequences or protospacer sequences. Spacer sequences may be 10, 11, 12, 13, 14, 15, 16, 17, 18, 19, 20, 21, 22, 23, 24, 25, 26, 27, 28, 29, 30, 31, 32, 33, 34, 35, or 36 nucleotides in length. In some examples, the spacer sequence may be less than 10 or more than 36 nucleotides in length.

A gRNA may comprise a repeat sequence. In some cases, the repeat sequence is part of a double stranded portion of the gRNA. A repeat sequence may be 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides in length. In some examples, the spacer sequence may be less than 10 or more than 50 nucleotides in length.

A gRNA may comprise one or more synthetic nucleotides, non-naturally occurring nucleotides, nucleotides with a modification, deoxyribonucleotide, or any combination thereof. Additionally or alternatively, a gRNA may comprise a hairpin, linker region, single stranded region, double stranded region, or any combination thereof. Additionally or alternatively, a gRNA may comprise a signaling or reporter molecule.

gRNAs may be encoded by genetic or episomal DNA. gRNAs may be provided or delivered concomitantly with a CRISPR nuclease or sequentially. Guide RNAs may be chemically synthesized, in vitro transcribed or otherwise generated using standard RNA generation techniques known in the art.

A CRISPR system may be a Type II CRISPR system, for example a Cas9 system. The Type II nuclease may comprise a single effector protein, which, in some cases, comprises a RuvC and HNH nuclease domains. In some cases a functional Type II nuclease may comprise two or more polypeptides, each of which comprises a nuclease domain or fragment thereof. The target nucleic acid sequences may comprise a 3' protospacer adjacent motif (PAM). In some examples, the PAM may be 5' of the target nucleic acid. Guide RNAs (gRNA) may comprise a single chimeric gRNA, which contains both crRNA and tracrRNA sequences. Alternatively, the gRNA may comprise a set of two RNAs, for example a crRNA and a tracrRNA. The Type II nuclease may generate a double strand break, which is some cases creates two blunt ends. In some cases, the Type II CRISPR nuclease is engineered to be a nickase such that the nuclease only generates a single strand break. In such cases, two distinct nucleic acid sequences may be targeted by gRNAs such that two single strand breaks are generated by the nickase. In some examples, the two single strand breaks effectively create a double strand break. In some cases where a Type II nickase is used to generate two single strand breaks, the resulting nucleic acid free ends may either be blunt, have a 3' overhang, or a 5' overhang. In some examples, a Type II nuclease may be catalytically dead such that it binds to a target sequence, but does not cleave. For example, a Type II nuclease may have mutations in both the RuvC and HNH domains, thereby rendering the both nuclease domains non-functional. A Type II CRISPR system may be one of three sub-types, namely Type II-A, Type II-B, or Type II-C.

A CRISPR system may be a Type V CRISPR system, for example a Cpf1, C2c1, or C2c3 system. The Type V nuclease may comprise a single effector protein, which in some cases comprises a single RuvC nuclease domain. In other cases, a function Type V nuclease comprises a RuvC domain split between two or more polypeptides. In such cases, the target nucleic acid sequences may comprise a 5' PAM or 3' PAM. Guide RNAs (gRNA) may comprise a single gRNA or single crRNA, such as may be the case with Cpf1. In some cases, a tracrRNA is not needed. In other examples, such as when C2c1 is used, a gRNA may comprise a single chimeric gRNA, which contains both crRNA and tracrRNA sequences or the gRNA may comprise a set of two RNAs, for example a crRNA and a tracrRNA. The Type V CRISPR nuclease may generate a double strand break, which in some cases generates a 5' overhang. In some cases, the Type V CRISPR nuclease is engineered to be a nickase such that the nuclease only generates a single strand break. In such cases, two distinct nucleic acid sequences may be targeted by gRNAs such that two single strand breaks are generated by the nickase. In some examples, the two single strand breaks effectively create a double strand break. In some cases where a Type V nickase is used to generate two single strand breaks, the resulting nucleic acid free ends may either be blunt, have a 3' overhang, or a 5' overhang. In some examples, a Type V nuclease may be catalytically dead such that it binds to a target sequence, but does not cleave. For example, a Type V nuclease could have mutations a RuvC domain, thereby rendering the nuclease domain non-functional.

A CRISPR system may be a Type VI CRISPR system, for example a C2c2 system. A Type VI nuclease may comprise a HEPN domain. In some examples, the Type VI nuclease comprises two or more polypeptides, each of which comprises a HEPN nuclease domain or fragment thereof. In such cases, the target nucleic acid sequences may by RNA, such as single stranded RNA. When using Type VI CRISPR system, a target nucleic acid may comprise a protospacer flanking site (PFS). The PFS may be 3' or 5'or the target or protospacer sequence. Guide RNAs (gRNA) may comprise a single gRNA or single crRNA. In some cases, a tracrRNA is not needed. In other examples, a gRNA may comprise a single chimeric gRNA, which contains both crRNA and tracrRNA sequences or the gRNA may comprise a set of two RNAs, for example a crRNA and a tracrRNA. In some examples, a Type VI nuclease may be catalytically dead such that it binds to a target sequence, but does not cleave. For example, a Type VI nuclease may have mutations in a HEPN domain, thereby rendering the nuclease domains non-functional.

Non-limiting examples of suitable nucleases, including nucleic acid-guided nucleases, for use in the present disclosure include C2c1, C2c2, C2c3, Cas1, Cas1B, Cas2, Cas3, Cas4, Cas5, Cas6, Cas7, Cas8, Cas9 (also known as Csn1 and Csx12), Cas10, Cpf1, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx100, Csx16, CsaX, Csx3, Csx1, Csx15, Csf1, Csf2, Csf3, Csf4, homologues thereof, orthologues thereof, or modified versions thereof.

In some methods disclosed herein, Argonaute (Ago) systems may be used to cleave target nucleic acid sequences. Ago protein may be derived from a prokaryote, eukaryote, or archaea. The target nucleic acid may be RNA or DNA. A DNA target may be single stranded or double stranded. In some examples, the target nucleic acid does not require a specific target flanking sequence, such as a sequence equivalent to a protospacer adjacent motif or protospacer flanking sequence. The Ago protein may create a double strand break or single strand break. In some examples, when a Ago protein forms a single strand break, two Ago proteins may be used in combination to generate a double strand break. In some examples, an Ago protein comprises one, two, or more nuclease domains. In some examples, an Ago protein comprises one, two, or more catalytic domains. One or more nuclease or catalytic domains may be mutated in the Ago protein, thereby generating a nickase protein capable of generating single strand breaks. In other examples, mutations in one or more nuclease or catalytic domains of an Ago protein generates a catalytically dead Ago protein that may bind but not cleave a target nucleic acid.

Ago proteins may be targeted to target nucleic acid sequences by a guiding nucleic acid. In many examples, the guiding nucleic acid is a guide DNA (gDNA). The gDNA may have a 5' phosphorylated end. The gDNA may be single stranded or double stranded. Single stranded gDNA may be 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides in length. In some examples, the gDNA may be less than 10 nucleotides in length. In some examples, the gDNA may be more than 50 nucleotides in length.

Argonaute-mediated cleavage may generate blunt end, 5' overhangs, or 3' overhangs. In some examples, one or more nucleotides are removed from the target site during or following cleavage.

Argonaute protein may be endogenously or recombinantly expressed. Argonaute may be encoded on a chromosome, extrachromosomally, or on a plasmid, synthetic chromosome, or artificial chromosome. Additionally or alternatively, an Argonaute protein may be provided as a polypeptide or mRNA encoding the polypeptide. In such examples, polypeptide or mRNA may be delivered through standard mechanisms known in the art, such as through the use of peptides, nanoparticles, or viral particles.

Guide DNAs may be provided by genetic or episomal DNA. In some examples, gDNA are reverse transcribed from RNA or mRNA. In some examples, guide DNAs may be provided or delivered concomitantly with an Ago protein or sequentially. Guide DNAs may be chemically synthesized, assembled, or otherwise generated using standard DNA generation techniques known in the art. Guide DNAs may be cleaved, released, or otherwise derived from genomic DNA, episomal DNA molecules, isolated nucleic acid molecules, or any other source of nucleic acid molecules.

Nuclease fusion proteins may be recombinantly expressed. A nuclease fusion protein may be encoded on a chromosome, extrachromosomally, or on a plasmid, synthetic chromosome, or artificial chromosome. A nuclease and a chromatin-remodeling enzyme may be engineered separately, and then covalently linked. A nuclease fusion protein may be provided as a polypeptide or mRNA encoding the polypeptide. In such examples, polypeptide or mRNA may be delivered through standard mechanisms known in the art, such as through the use of peptides, nanoparticles, or viral particles.

A guide nucleic acid may complex with a compatible nucleic acid-guided nuclease and may hybridize with a target sequence, thereby directing the nuclease to the target sequence. A subject nucleic acid-guided nuclease capable of complexing with a guide nucleic acid may be referred to as a nucleic acid-guided nuclease that is compatible with the guide nucleic acid. Likewise, a guide nucleic acid capable of complexing with a nucleic acid-guided nuclease may be referred to as a guide nucleic acid that is compatible with the nucleic acid-guided nucleases.

A guide nucleic acid may be DNA. A guide nucleic acid may be RNA. A guide nucleic acid may comprise both DNA and RNA. A guide nucleic acid may comprise modified of non-naturally occurring nucleotides. In cases where the guide nucleic acid comprises RNA, the RNA guide nucleic acid may be encoded by a DNA sequence on a polynucleotide molecule such as a plasmid, linear construct, or editing cassette as disclosed herein.

A guide nucleic acid may comprise a guide sequence. A guide sequence is a polynucleotide sequence having sufficient complementarity with a target polynucleotide sequence to hybridize with the target sequence and direct sequence-specific binding of a complexed nucleic acid-guided nuclease to the target sequence. The degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences. In some aspects, a guide sequence is about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length. In some aspects, a guide sequence is less than about 75, 50, 45, 40, 35, 30, 25, 20 nucleotides in length. Preferably the guide sequence is 10-30 nucleotides long. The guide sequence may be 10-25 nucleotides in length. The guide sequence may be 10-20 nucleotides in length. The guide sequence may be 15-30 nucleotides in length. The guide sequence may be 20-30 nucleotides in length. The guide sequence may be 15-25 nucleotides in length. The guide sequence may be 15-20 nucleotides in length. The guide sequence may be 20-25 nucleotides in length. The guide sequence may be 22-25 nucleotides in length. The guide sequence may be 15 nucleotides in length. The guide sequence may be 16 nucleotides in length. The guide sequence may be 17 nucleotides in length. The guide sequence may be 18 nucleotides in length. The guide sequence may be 19 nucleotides in length. The guide sequence may be 20 nucleotides in length. The guide sequence may be 21 nucleotides in length. The guide sequence may be 22 nucleotides in length. The guide sequence may be 23 nucleotides in length. The guide sequence may be 24 nucleotides in length. The guide sequence may be 25 nucleotides in length.

A guide nucleic acid may comprise a scaffold sequence. In general, a "scaffold sequence" includes any sequence that has sufficient sequence to promote formation of a targetable nuclease complex, wherein the targetable nuclease complex comprises a nucleic acid-guided nuclease and a guide nucleic acid comprising a scaffold sequence and a guide sequence. Sufficient sequence within the scaffold sequence to promote formation of a targetable nuclease complex may include a degree of complementarity along the length of two sequence regions within the scaffold sequence, such as one or two sequence regions involved in forming a secondary structure. In some cases, the one or two sequence regions are comprised or encoded on the same polynucleotide. In some cases, the one or two sequence regions are comprised or encoded on separate polynucleotides. Optimal alignment may be determined by any suitable alignment algorithm, and may further account for secondary structures, such as self-complementarity within either the one or two sequence regions. In some aspects, the degree of complementarity between the one or two sequence regions along the length of the shorter of the two when optimally aligned is about or more than about 25%, 30%, 40%, 50%, 60%, 70%, 80%, 90%, 95%, 97.5%, 99%, or higher. In some aspects, at least one of the two sequence regions is about or more than about 5, 6, 7, 8, 9, 10, 11, 12, 13, 14, 15, 16, 17, 18, 19, 20, 21, 22, 23, 24, 25, 30, 40, 50, or more nucleotides in length. In some aspects, at least one of the two sequence regions is about 10-30 nucleotides in length. At least one of the two sequence regions may be 10-25 nucleotides in length. At least one of the two sequence regions may be 10-20 nucleotides in length. At least one of the two sequence regions may be 15-30 nucleotides in length. At least one of the two sequence regions may be 20-30 nucleotides in length. At least one of the two sequence regions may be 15-25 nucleotides in length. At least one of the two sequence regions may be 15-20 nucleotides in length. At least one of the two sequence regions may be 20-25 nucleotides in length. At least one of the two sequence regions may be 22-25 nucleotides in length. At least one of the two sequence regions may be 15 nucleotides in length. At least one of the two sequence regions may be 16 nucleotides in length. At least one of the two sequence regions may be 17 nucleotides in length. At least one of the two sequence regions may be 18 nucleotides in length. At least one of the two sequence regions may be 19 nucleotides in length. At least one of the two sequence regions may be 20 nucleotides in length. At least one of the two sequence regions may be 21 nucleotides in length. At least one of the two sequence regions may be 22 nucleotides in length. At least one of the two sequence regions may be 23 nucleotides in length. At least one of the two sequence regions may be 24 nucleotides in length. At least one of the two sequence regions may be 25 nucleotides in length.

A scaffold sequence of a subject guide nucleic acid may comprise a secondary structure. A secondary structure may comprise a pseudoknot region. In some example, the compatibility of a guide nucleic acid and nucleic acid-guided nuclease is at least partially determined by sequence within or adjacent to a pseudoknot region of the guide RNA. In some cases, binding kinetics of a guide nucleic acid to a nucleic acid-guided nuclease is determined in part by secondary structures within the scaffold sequence. In some cases, binding kinetics of a guide nucleic acid to a nucleic acid-guided nuclease is determined in part by nucleic acid sequence with the scaffold sequence.

In aspects of the disclosure the terms "guide nucleic acid" refers to a polynucleotide comprising 1) a guide sequence capable of hybridizing to a target sequence and 2) a scaffold sequence capable of interacting with or complexing with a nucleic acid-guided nuclease as described herein.

A guide nucleic acid may be compatible with a nucleic acid-guided nuclease when the two elements may form a functional targetable nuclease complex capable of cleaving a target sequence. Often, a compatible scaffold sequence for a compatible guide nucleic acid may be found by scanning sequences adjacent to native nucleic acid-guided nuclease loci. In other words, native nucleic acid-guided nucleases may be encoded on a genome within proximity to a corresponding compatible guide nucleic acid or scaffold sequence.

Nucleic acid-guided nucleases may be compatible with guide nucleic acids that are not found within the nucleases endogenous host. Such orthogonal guide nucleic acids may be determined by empirical testing. Orthogonal guide nucleic acids may come from different bacterial species or be synthetic or otherwise engineered to be non-naturally occurring.

Orthogonal guide nucleic acids that are compatible with a common nucleic acid-guided nuclease may comprise one or more common features. Common features may include sequence outside a pseudoknot region. Common features may include a pseudoknot region. Common features may include a primary sequence or secondary structure.

A guide nucleic acid may be engineered to target a desired target sequence by altering the guide sequence such that the guide sequence is complementary to the target sequence, thereby allowing hybridization between the guide sequence and the target sequence. A guide nucleic acid with an engineered guide sequence may be referred to as an engineered guide nucleic acid. Engineered guide nucleic acids are often non-naturally occurring and are not found in nature.

A guide RNA molecule comprises sequence that base-pairs with target sequence that is to be isolated for sequencing. In some embodiments the base-pairing is complete, while in some embodiments the base pairing is partial or comprises bases that are unpaired along with bases that are paired to nontarget sequence.

A guide RNA may comprise a region or regions that form an RNA 'hairpin' structure. Such region or regions comprise partially or completely palindromic sequence, such that 5' and 3' ends of the region may hybridize to one another to form a double-strand 'stem' structure, which in some embodiments is capped by a non-palindromic loop tethering each of the single strands in the double strand loop to one another.

In some embodiments the Guide RNA comprises a stem loop such as a tracrRNA stem loop. A stem loop such as a tracrRNA stem loop may complex with or bind to a nucleic acid endonuclease such as Cas9 DNA endonuclease. Alternately, a stem loop may complex with an endonuclease other than Cas9 or with a nucleic acid modifying enzyme other than an endonuclease, such as a base excision enzyme, a methyltransferase, or an enzyme having other nucleic acid modifying activity that interferes with one or more DNA polymerase enzymes.

The tracrRNA / CRISPR / Endonuclease system was identified as an adaptive immune system in eubacterial and archaeal prokaryotes whereby cells gain resistance to repeated infection by a virus of a known sequence. See, for example, Deltcheva E, Chylinski K, Sharma CM, Gonzales K, Chao Y, Pirzada ZA et al. (2011) "CRISPR RNA maturation by trans-encoded small RNA and host factor RNase III" Nature 471 (7340): 602-7. doi:10.1038/nature09886. PMC 3070239. PMID 21455174; Terns MP, Terns RM (2011) "CRISPR-based adaptive immune systems" Curr Opin Microbiol 14 (3): 321-7. doi:10.1016/j.mib.2011.03.005. PMC 3119747. PMID 21531607; Jinek M, Chylinski K, Fonfara I, Hauer M, Doudna JA, Charpentier E (2012) "A Programmable Dual-RNA-Guided DNA Endonuclease in Adaptive Bacterial Immunity" Science 337 (6096): 816-21. doi:10.1126/science.1225829. PMID 22745249; and Brouns SJ (2012) "A swiss army knife of immunity" Science 337 (6096): 808-9. doi:10.1126/science.1227253. PMID 22904002. The system has been adapted to direct targeted mutagenesis in eukaryotic cells. See, e.g., Wenzhi Jiang, Huanbin Zhou, Honghao Bi, Michael Fromm, Bing Yang, and Donald P. Weeks (2013) "Demonstration of CRISPR/Cas9/sgRNA-mediated targeted gene modification in Arabidopsis, tobacco, sorghum and rice" Nucleic Acids Res. Nov 2013; 41(20): e188, Published online Aug 31, 2013. doi: 10.1093/nar/gkt780, and references therein.

As contemplated herein, guide RNA are used in some embodiments to provide sequence specificity to a DNA endonuclease such as a Cas9 endonuclease. In these embodiments a guide RNA comprises a hairpin structure that binds to or is bound by an endonuclease such as Cas9 (other endonucleases are contemplated as alternatives or additions in some embodiments), and a guide RNA further comprises a recognition sequence that binds to or specifically binds to or exclusively binds to a sequence that is to be removed from a sequencing library or a sequencing reaction. The length of the recognition sequence in a guide RNA may vary according to the degree of specificity desired in the sequence elimination process. Short recognition sequences, comprising frequently occurring sequence in the sample or comprising differentially abundant sequence (abundance of AT in an AT-rich genome sample or abundance of GC in a GC-rich genome sample) are likely to identify a relatively large number of sites and therefore to direct frequent nucleic acid modification such as endonuclease activity, base excision, methylation or other activity that interferes with at least one DNA polymerase activity. Long recognition sequences, comprising infrequently occurring sequence in the sample or comprising underrepresented base combinations (abundance of GC in an AT-rich genome sample or abundance of AT in a GC-rich genome sample) are likely to identify a relatively small number of sites and therefore to direct infrequent nucleic acid modification such as endonuclease activity, base excision, methylation or other activity that interferes with at least one DNA polymerase activity. Accordingly, as disclosed herein, in some embodiments one may regulate the frequency of sequence removal from a sequence reaction through modifications to the length or content of the recognition sequence.

Guide RNA may be synthesized through a number of methods consistent with the disclosure herein. Standard synthesis techniques may be used to produce massive quantities of guide RNAs, and/or for highly-repetitive targeted regions, which may require only a few guide RNA molecules to target a multitude of unwanted loci. The double stranded DNA molecules can comprise an RNA site specific binding sequence, a guide RNA sequence for Cas9 protein and a T7 promoter site. In some cases, the double stranded DNA molecules can be less than about 100bp length. T7 polymerase can be used to create the single stranded RNA molecules, which may include the target RNA sequence and the guide RNA sequence for the Cas9 protein.

Guide RNA sequences may be designed through a number of methods. For example, in some embodiments, non-genic repeat sequences of the human genome are broken up into, for example, 100bp sliding windows. Double stranded DNA molecules can be synthesized in parallel on a microarray using photolithography.

The windows may vary in size. 30-mer target sequences can be designed with a short trinucleotide protospacer adjacent motif (PAM) sequence of N-G-G flanking the 5' end of the target design sequence, which in some cases facilitates cleavage. See, among others, Giedrius Gasiunas et al.,

(2012) "Cas9-crRNA ribonucleoprotein complex mediates specific DNA cleavage for adaptive immunity in bacteria" Proc. Natl. Acad. Sci. USA. Sep 25, 109(39): E2579-E2586, which is hereby incorporated by reference in its entirety. Redundant sequences can be eliminated and the remaining sequences can be analyzed using a search engine (e.g. BLAST) against the human genome to avoid hybridization against refseq, ENSEMBL and other gene databases to avoid nuclease activity at these sites. The universal Cas9 tracer RNA sequence can be added to the guide RNA target sequence and then flanked by the T7 promoter. The sequences upstream of the T7 promoter site can be synthesized. Due to the highly repetitive nature of the target regions in the human genome, in many embodiments, a relatively small number of guide RNA molecules will digest a larger percentage of NGS library molecules.

Although only about 50% of protein coding genes are estimated to have exons comprising the NGG PAM (photospacer adjacent motif) sequence, multiple strategies are provided herein to increase the percentage of the genome that can be targeted with the Cas9 cutting system. For example, if a PAM sequence is not available in a DNA region, a PAM sequence may be introduced via a combination strategy using a guide RNA coupled with a helper DNA comprising the PAM sequence. The helper DNA can be synthetic and/or single stranded. The PAM sequence in the helper DNA will not be complimentary to the gDNA knockout target in the NGS library, and may therefore be unbound to the target NGS library template, but it can be bound to the guide RNA. The guide RNA can be designed to hybridize to both the target sequence and the helper DNA comprising the PAM sequence to form a hybrid DNA:RNA:DNA complex that can be recognized by the Cas9 system.

The PAM sequence may be represented as a single stranded overhang or a hairpin. The hairpin can, in some cases, comprise modified nucleotides that may optionally be degraded. For example, the hairpin can comprise Uracil, which can be degraded by Uracil DNA Glycosylase.

As an alternative to using a DNA comprising a PAM sequence, modified Cas9 proteins without the need of a PAM sequence or modified Cas9 with lower sensitivity to PAM sequences may be used without the need for a helper DNA sequence.

In further cases, the guide RNA sequence used for Cas9 recognition may be lengthened and inverted at one end to act as a dual cutting system for close cutting at multiple sites. The guide RNA sequence can produce two cuts on a NGS DNA library target. This can be achieved by designing a single guide RNA to alternate strands within a restricted distance. One end of the guide RNA may bind to the forward strand of a double stranded DNA library and the other may bind to the reverse strand. Each end of the guide RNA can comprise the PAM sequence and a Cas9 binding domain. This may result in a dual double stranded cut of the NGS library molecules from the same DNA sequence at a defined distance apart. Some embodiments relate to the generation of guide RNA molecules. Guide RNA molecules are in some cases transcribed from DNA templates. A number of RNA polymerases may be used, such as T7 polymerase, RNA PolI, RNA PolII, RNA PolIII, an organellar RNA polymerase, a viral RNA polymerase, or a eubacterial or archaeal polymerase. In some cases the polymerase is T7.

Guide RNA generating templates comprise a promoter, such as a promoter compatible with transcription directed by T7 polymerase, RNA PolI, RNA PolII, RNA PolIII, an organellar RNA polymerase, a viral RNA polymerase, or a eubacterial or archaeal polymerase. In some cases the promoter is a T7 promoter.

Guide RNA templates encode a tag sequence in some cases. A tag sequence binds to a nucleic acid modifying enzyme such as a methylase, base excision enzyme or an endonuclease. In the context of a larger Guide RNA molecule bound to a nontarget site, a tag sequence tethers an enzyme to a nucleic acid nontarget region, directing activity to the nontarget site. An exemplary tethered enzyme is an endonuclease such as Cas9.

Guide RNA templates are complementary to the nucleic acid corresponding to ribosomal RNA sequences, sequences encoding globin proteins, sequences encoding a transposon, sequences encoding retroviral sequences, sequences comprising telomere sequences, sequences comprising sub-telomeric repeats, sequences comprising centromeric sequences, sequences comprising intron sequences, sequences comprising Alu repeats, sequences comprising SINE repeats, sequences comprising LINE repeats, sequences comprising dinucleic acid repeats, sequences comprising trinucleic acid repeats, sequences comprising tetranucleic acid repeats, sequences comprising poly-A repeats, sequences comprising poly-T repeats, sequences comprising poly-C repeats, sequences comprising poly-G repeats, sequences comprising AT -rich sequences, or sequences comprising GC-rich sequences.

In many cases, the tag sequence comprises a stem-loop, such as a partial or total stem-loop structure. The 'stem' of the stem loop structure is encoded by a palindromic sequence in some cases, either complete or interrupted to introduce at least one 'kink' or turn in the stem. The 'loop' of the stem loop structure is not involved in stem base pairing in most cases. In some cases, the stem loop is encoded by a tracr sequence. Some stem loops bind, for example, Cas9 or other endonuclease.

Guide RNA molecules additionally comprise a recognition sequence. The recognition sequence is completely or incompletely reverse-complementary to a nontarget sequence to be eliminated from a nucleic acid library sequence set. As RNA is able to hybridize using base pair combinations (G:U base pairing, for example) that do not occur in DNA-DNA hybrids, the recognition sequence does not need to be an exact reverse complement of the nontarget sequence to bind. In addition, small perturbations from complete base pairing are tolerated in some cases.

### EXAMPLES

The following examples are given for the purpose of illustrating various embodiments of the invention and are not meant to limit the present invention in any fashion. The present examples, along with the methods described herein are presently representative of preferred embodiments, are exemplary, and are not intended as limitations on the scope of the invention.

### Example 1: Targeted Sequencing with CRISPR Cas9 in B Reaction

A primer extension reaction is conducted with barcoded random primers that do not have adapter sequences. The primer extension products are terminated with biotin-ddNTPs and bound to streptavidin beads. The random B primer is used to generate a complementary strand. Cas9-guide RNA complexes are designed to target specific loci of interest and added to the DNA bound to the streptavidin beads. Cas9-guide RNA complexes cleave the DNA at the target sites. Released DNA is transferred to a new tube. Any NGS library preparation protocol involving adapter ligation is used to prepare DNA for sequencing. This example is illustrated in **FIG. 1****.**

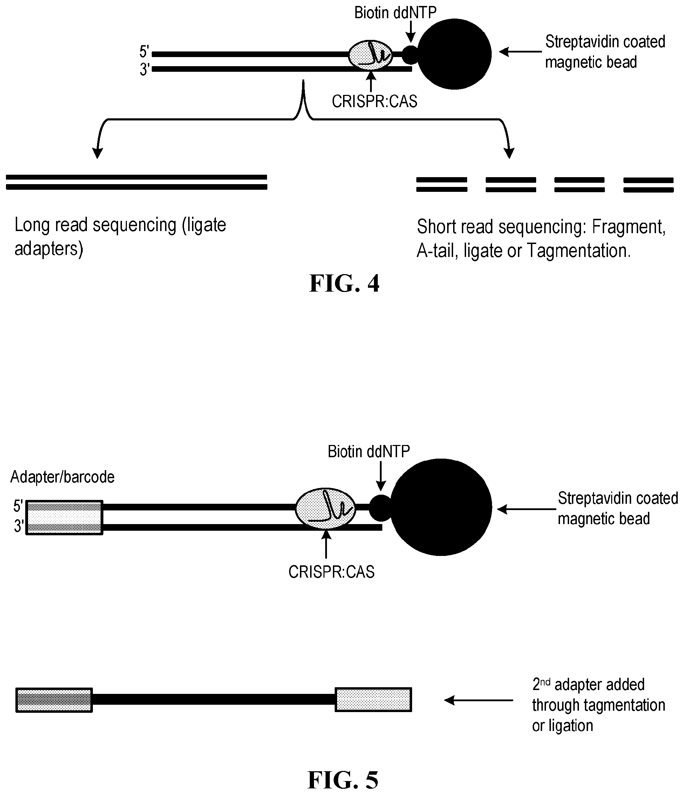

In cases where long read sequencing is used, adapters are ligated directly onto purified product. In cases where short read sequencing is used, the sample is fragmented, polyadenylated, ligated, or tagmentation is used. These options are illustrated in **FIG. 4****.**

An adapter/barcode are ligated onto the sample while still bead-bound (i.e., before CRISPR reaction takes place). A second adapter/barcode is added after cleavage using tagmentation or ligation. This is illustrated in **FIG. 5****.**

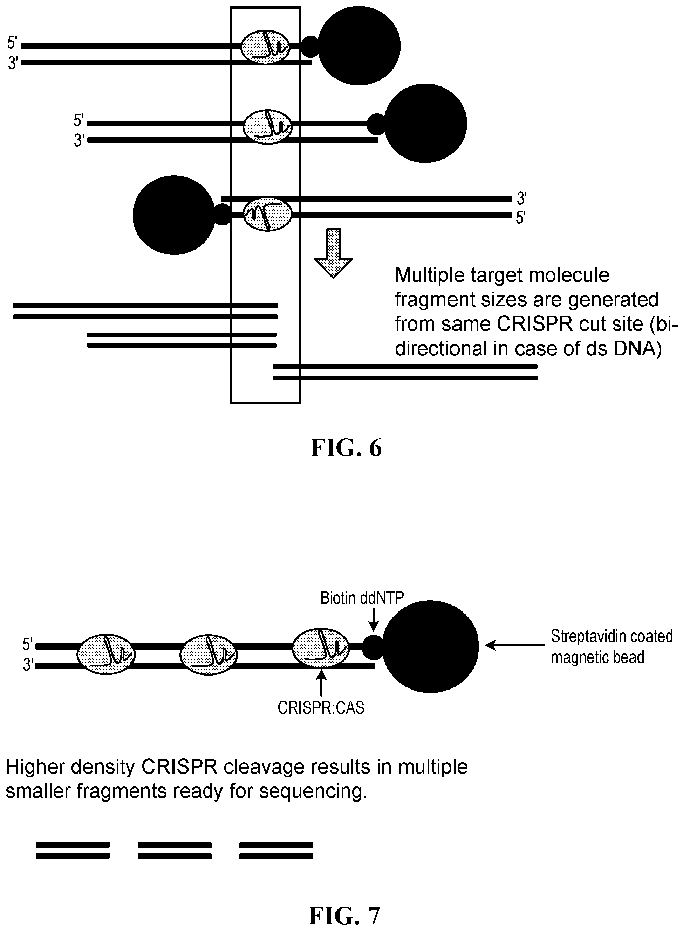

Multiple target molecules sizes are obtained using a CRISPR cut site. This is illustrated in **FIG. 6****.** If shorter fragments are desired, higher density CRISPR cleavage is designed **(****FIG. 7****).**

### Example 2: Targeted Sequencing of COVID-19

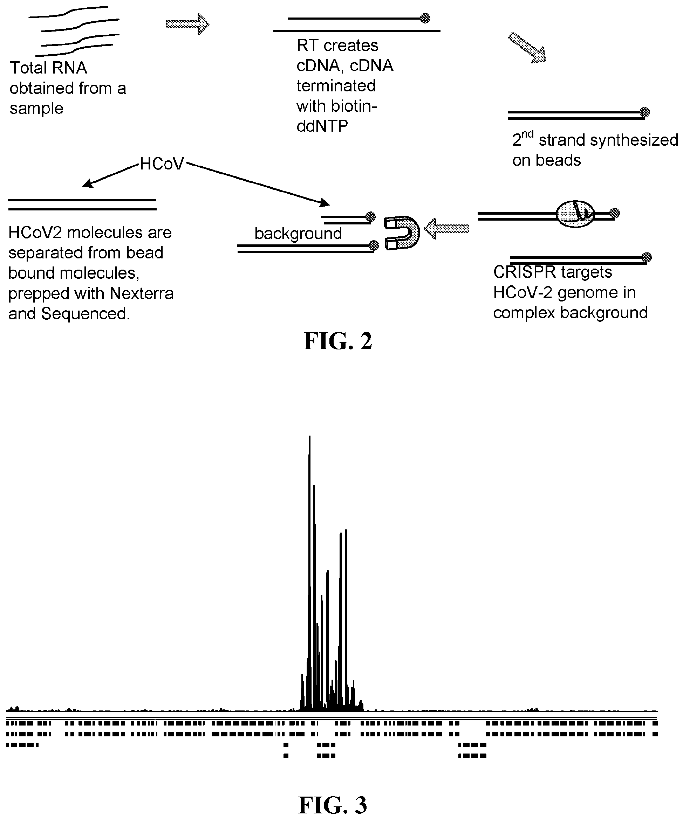

Total RNA from a patient sample is obtained and reverse transcribed using reverse transcriptase to make cDNA terminated with a biotinylated dideoxynucleotidet triphosphate (ddNTP) and captured on a streptavidin magnetic bead. A second strand is synthesized on the beads. A CRISPR complex specific for HCoV-2 genome targets the COVID-19 nucleic acids in the sample. The background nucleic acids are removed from the sample using a magnet leaving purified HCoV2 nucleic acid molecules that are then prepared using Nexterra for sequencing. This example is illustrated in **FIG. 2****.**

### Example 3: Targeted Sequencing of E. coli genome

A biological sample comprising E. *coli* genomic DNA was obtained. A primer extension reaction was performed with barcoded random primers without Illumina adapter. Primer-extended molecules were captured with streptavidin magnetic beads. Beads were washed beads three times to remove non-specifically bound DNA molecules. A primer extension reaction was performed with barcoded random primers without Illumina adapter to create complementary strand. Beads were washed three times to remove non-specifically bound DNA molecules. Beads were resuspended in 1X Cas9 buffer. Pre-complexed Cas9:sgRNA was added to the streptavidin beads (sgRNA pool contains guide RNAs targeting all E. *coli* loci). The reaction was incubated at 37 C for 30 - 60 mins for Cas9-mediated site-specific cleavage to occur. Clipped molecules were no longer bound to beads. The tube was placed on magnetic stand and supernatant transferred to new tube. The new tube was placed back on the magnetic stand and supernatant was transferred to a new tube to get rid of residual beads. Standard end-repair, phosphorylation and A-tailing reaction were performed using reagents from the NEBNext Ultra II DNA Library Prep Kit (using the NEB protocol). Illumina adapter ligation was performed using reagents from the NEBNext Ultra II DNA Library Prep Kit (using the NEB protocol). 0.9X Ampure bead cleanup was performed (using the NEB protocol). PCR amplification was performed with full length Illumina P5 and P7 primers (using the NEB protocol). Final Ampure bead cleanup was performed (using the NEB protocol). Sequencing of the purified amplification product was performed. The data from this experiment is illustrated in **FIG. 3****.**

## Claims

1. A method of creating a nucleic acid library comprising a target nucleic acid, the method comprising:
(a) contacting a nucleic acid sample to a first population of primers, a polymerase, dNTPs, and labeled ddNTPs;
(b) performing an extension reaction thereby creating an labeled extension product;
(c) contacting the extension product to a second population of primers to create a double stranded extension product comprising the target nucleic acid;
(d) contacting the double stranded extension product to a target specific enzyme under conditions allowing cleavage of at least a subset of the double stranded extension product thereby creating a cleaved target nucleic acid; and
(e) isolating the cleaved target nucleic acid, thereby creating a nucleic acid library.

2. The method of claim 1, wherein the nucleic acid sample is denatured.

3. The method of claim 1 or claim 2, wherein the first or second population of primers comprise random primers or oligo-dT primers, and optionally wherein the first or second population of primers comprise a barcode.

4. The method of any one of claims 1 to 3, wherein the labeled ddNTPs comprise biotin.

5. The method of any one of claims 1 to 4, wherein (i) the labeled extension product is isolated on a bead, wherein the bead is a streptavidin bead or a magnetic bead or, wherein (ii) the labeled extension product is isolated on a polypropylene, or a polycarbonate surface.

6. The method of any one of claims 1 to 5, wherein the target specific enzyme is complexed with a target specific guide RNA, optionally wherein the target specific enzyme is a Cas nuclease, and optionally wherein the Cas nuclease is a Cas9 nuclease.

7. The method of any one of claims 1 to 5, wherein the target specific enzyme is a nuclease, and optionally wherein the nuclease is a restriction endonuclease.

8. The method of any one of claims 1 to 7, wherein the target nucleic acid is a deoxyribonucleic acid or a ribonucleic acid and wherein the polymerase is a DNA polymerase, an RNA polymerase, a reverse transcriptase, or a combination thereof.

9. A method of generating a sequencing library comprising a target nucleic acid sequence, the method comprising: (a) incorporating an affinity molecule to a double stranded DNA molecule comprising a target nucleic acid sequence thereby obtaining an affinity molecule-coupled double stranded DNA molecule, (b) capturing the affinity molecule coupled double stranded DNA molecule using a binding agent that binds to the affinity molecule; (c) cutting the target nucleic acid using a CRISPR enzyme mediated cleavage, and (d) isolating the cleaved target nucleic acid from the solid substrate and converting into a sequencing library.

10. The method of claim 9, wherein the incorporating an affinity molecule is achieved by ligation or by conjugation chemistry.

11. The method of claim 9, wherein the capturing comprises capturing the affinity molecule coupled double stranded DNA molecule on a solid substrate, optionally wherein the solid substrate is a streptavidin bead.

12. The method of any one of claims 9 to 11, wherein the affinity molecule is biotin, and the binding agent is streptavidin.

13. The method of any one of claims 9 to 12, wherein cutting the target nucleic acid comprises cleavage of the DNA at target sites by Cas9-guide RNA complexes that target specific loci.

14. The method of claim 9, wherein isolating comprises eluting the double-stranded target nucleic acid from the solid substrate.

15. The method of any one of claims 1 to 14, wherein the target nucleic acid comprises a viral nucleic acid, a bacterial nucleic acid, a pathogen nucleic acid, a single nucleotide polymorphism, a disease associated gene, a cancer associated gene, a human leukocyte antigen gene, or a combination thereof, wherein the viral nucleic acid is from an influenza virus, a coronavirus, a rhinovirus, a herpesvirus, or a human immunodeficiency virus, and wherein optionally the coronavirus is a COVID-19 virus.

## Patentansprüche

1. Verfahren zum Erstellen einer Nukleinsäurebibliothek, die eine Zielnukleinsäure umfasst, wobei das Verfahren umfasst:
(a) Inberührungbringen einer Nukleinsäureprobe mit einer ersten Population von Primern, einer Polymerase, dNTPs, und markierten ddNTPs;
(b) Durchführen einer Verlängerungsreaktion, wodurch ein markiertes Verlängerungsprodukt erstellt wird;
(c) Inberührungbringen des Verlängerungsprodukts mit einer zweiten Population von Primern, um ein doppelsträngiges Verlängerungsprodukt zu erstellen, das die Zielnukleinsäure umfasst;
(d) Inberührungbringen des doppelsträngigen Verlängerungsprodukts mit einem zielspezifischen Enzym unter Bedingungen, die die Spaltung wenigstens einer Teilmenge des doppelsträngigen Verlängerungsprodukts ermöglichen, wodurch eine gespaltene Zielnukleinsäure erstellt wird; und
(e) Isolieren der gespaltenen Zielnukleinsäure, wodurch eine Nukleinsäurebibliothek erstellt wird.

2. Verfahren nach Anspruch 1, wobei die Nukleinsäureprobe denaturiert wird.

3. Verfahren nach Anspruch 1 oder 2, wobei die erste oder die zweite Population von Primern Primer oder Oligo-dT-Primer umfasst und wobei optional die erste oder die zweite Population von Primern einen Barcode umfasst.

4. Verfahren nach einem der Ansprüche 1 bis 3, wobei die markierten ddNTPs Biotin umfassen.

5. Verfahren nach einem der Ansprüche 1 bis 4, wobei (i) das markierte Verlängerungsprodukt auf einem Kügelchen isoliert wird, wobei das Kügelchen ein Streptavidinkügelchen oder ein magnetisches Kügelchen ist, oder wobei (ii) das markierte Verlängerungsprodukt auf einer Polypropylen- oder Polycarbonatoberfläche isoliert ist.

6. Verfahren nach einem der Ansprüche 1 bis 5, wobei das zielspezifische Enzym mit einer zielspezifischen Führungs-RNA komplexiert ist, optional wobei das zielspezifische Enzym eine Cas-Nuklease ist und optional wobei die Cas-Nuklease eine Cas9-Nuklease ist.

7. Verfahren nach einem der Ansprüche 1 bis 5, wobei das zielspezifische Enzym eine Nuklease ist, und optional wobei die Nuklease eine Restriktionsendonuklease ist.

8. Verfahren nach einem der Ansprüche 1 bis 7, wobei die Zielnukleinsäure eine Desoxyribonukleinsäure oder eine Ribonukleinsäure ist, und wobei die Polymerase eine DNA-Polymerase, eine RNA-Polymerase, eine reverse Transkriptase oder eine Kombination davon ist.

9. Verfahren zum Erzeugen einer Sequenzierungsbibliothek, die eine Zielnukleinsäuresequenz umfasst, wobei das Verfahren umfasst: (a) Einbauen eines Affinitätsmoleküls in ein doppelsträngiges DNA-Molekül, umfassend eine Zielnukleinsäuresequenz, wodurch ein affinitätsmolekülgekoppeltes doppelsträngiges DNA-Molekül erhalten wird, (b) Einfangen des affinitätsmolekülgekoppelten doppelsträngigen DNA-Moleküls unter Verwendung eines Bindemittels, das sich an das Affinitätsmolekül bindet; (c) Schneiden der Zielnukleinsäure unter Verwendung einer CRISPR-Enzymvermittelten Spaltung, und (d) Isolieren der gespaltenen Zielnukleinsäure aus dem festen Substrat und Umwandeln in eine Sequenzierungsbibliothek.

10. Verfahren nach Anspruch 9, wobei das Einbauen eines Affinitätsmoleküls durch Ligation oder durch Konjugationschemie erreicht wird.

11. Verfahren nach Anspruch 9, wobei das Einfangen das Einfangen des affinitätsmolekülgekoppelten doppelsträngigen DNA-Moleküls auf einem festen Substrat umfasst, optional wobei das feste Substrat ein Streptavidinkügelchen ist.

12. Verfahren nach einem der Ansprüche 9 bis 11, wobei das Affinitätsmolekül Biotin ist und das Bindemittel Streptavidin ist.

13. Verfahren nach einem der Ansprüche 9 bis 12, wobei das Schneiden der Zielnukleinsäure das Schneiden der DNA an Zielstellen durch Cas9-Führungs-RNA-Komplexe umfasst, die auf spezifische Loci abzielen.

14. Verfahren nach Anspruch 9, wobei das Isolieren das Eluieren der doppelsträngigen Zielnukleinsäure von dem festen Substrat umfasst.

15. Verfahren nach einem der Ansprüche 1 bis 14, wobei die Zielnukleinsäure eine virale Nukleinsäure, eine bakterielle Nukleinsäure, eine pathogene Nukleinsäure, einen Einzelnukleotidpolymorphismus, ein krankheitsassoziiertes Gen, ein krebsassoziiertes Gen, ein menschliches Leukozyten-Antigen-Gen oder eine Kombination davon umfasst, wobei die virale Nukleinsäure von einem Influenza-Virus, einem Coronavirus, einem Rhinovirus, einem Herpesvirus oder einem menschlichen Immunschwächevirus ist, optional wobei das Coronavirus ein COVID-19-Virus ist.

## Revendications

1. Procédé de création d'une banque d'acide nucléique comprenant un acide nucléique cible, le procédé comprenant :
(a) la mise en contact d'un échantillon d'acide nucléique avec une première population d'amorces, une polymérase, des dNTPs, et des ddNTPs marqués ;
(b) la réalisation d'une réaction d'extension, créant ainsi un produit d'extension marqué ;
(c) la mise en contact du produit d'extension avec une seconde population d'amorces pour créer un produit d'extension à double brin comprenant l'acide nucléique cible ;
(d) la mise en contact du produit d'extension à double brin avec un enzyme spécifique cible dans des conditions permettant le clivage d'au moins un sous-ensemble du produit d'extension à double brin, créant ainsi un acide nucléique cible clivé ; et
(e) l'isolement de l'acide nucléique cible clivé, créant ainsi une banque d'acide nucléique.

2. Procédé de la revendication 1, dans lequel l'échantillon d'acide nucléique est dénaturé.

3. Procédé de la revendication 1 ou la revendication 2, dans lequel la première ou seconde population d'amorces comprend des amorces aléatoires ou des amorces oligo-dT, et facultativement dans lequel la première ou seconde population d'amorces comprend un code à barres.

4. Procédé de l'une quelconque des revendications 1 à 3, dans lequel les ddNTPs marqués comprennent de la biotine.

5. Procédé de l'une quelconque des revendications 1 à 4, dans lequel (i) le produit d'extension marqué est isolé sur une bille, dans lequel la bille est une bille de streptavidine ou une bille magnétique ou, dans lequel (ii) le produit d'extension marqué est isolé sur un polypropylène, ou une surface de polycarbonate.

6. Procédé de l'une quelconque des revendications 1 à 5, dans lequel l'enzyme spécifique cible est complexé avec un ARN guide spécifique cible, facultativement dans lequel l'enzyme spécifique cible est une nucléase Cas, et facultativement dans lequel la nucléase Cas est une nucléase Cas9.

7. Procédé de l'une quelconque des revendications 1 à 5, dans lequel l'enzyme spécifique cible est une nucléase, et facultativement dans lequel la nucléase est une endonucléase de restriction.

8. Procédé de l'une quelconque des revendications 1 à 7, dans lequel l'acide nucléique cible est un acide désoxyribonucléique ou un acide ribonucléique et dans lequel la polymérase est une ADN polymérase, une ARN polymérase, une transcriptase inverse, ou une combinaison de celles-ci.

9. Procédé de génération d'une banque de séquençage comprenant une séquence d'acide nucléique cible, le procédé comprenant : (a) l'incorporation d'une molécule d'affinité dans une molécule d'ADN à double brin comprenant une séquence d'acide nucléique cible, obtenant ainsi une molécule d'ADN à double brin couplée avec molécule d'affinité, (b) la capture de la molécule d'ADN à double brin couplée avec molécule d'affinité en utilisant un agent liant qui se lie à la molécule d'affinité ; (c) la découpe de l'acide nucléique cible en utilisant un clivage à médiation par enzyme CRISPR, et (d) l'isolement, du substrat solide, de l'acide nucléique cible clivé, et la conversion en une banque de séquençage.

10. Procédé de la revendication 9, dans lequel l'incorporation d'une molécule d'affinité est accomplie par ligation ou par chimie de conjugaison.

11. Procédé de la revendication 9, dans lequel la capture comprend la capture de la molécule d'ADN à double brin couplée avec molécule d'affinité sur un substrat solide, facultativement dans lequel le substrat solide est une bille de streptavidine.

12. Procédé de l'une quelconque des revendications 9 à 11, dans lequel la molécule d'affinité est la biotine, et l'agent liant est la streptavidine.

13. Procédé de l'une quelconque des revendications 9 à 12, dans lequel la découpe de l'acide nucléique cible comprend le clivage de l'ADN à des sites cibles par des complexes Cas9-ARN guide qui ciblent des loci spécifiques.

14. Procédé de la revendication 9, dans lequel l'isolement comprend l'élution, du substrat solide, de l'acide nucléique cible à double brin.

15. Procédé de l'une quelconque des revendications 1 à 14, dans lequel l'acide nucléique cible comprend un acide nucléique viral, un acide nucléique bactérien, un acide nucléique pathogène, un polymorphisme mononucléotide, un gène associé à une maladie, un gène associé à un cancer, un gène antigène leucocytaire humain, ou une combinaison de ceux-ci, dans lequel l'acide nucléique viral provient d'un virus influenza, d'un coronavirus, d'un rhinovirus, d'un virus herpès, ou d'un virus d'immunodéficience humaine, et dans lequel facultativement le coronavirus est un virus COVID-19.
